# EUROPÄISCHE PATENTSCHRIFT

(11) **EP 0 644 883 B1**
(45) Veröffentlichungstag und Bekanntmachung des Hinweises auf die Patenterteilung: **15.09.1999**
(21) Anmeldenummer: 93912908.6
(22) Anmeldetag: 07.06.1993
(51) Int. Cl.: C07D 273/00, A61K 31/395, C07C 231/02, C07C 235/12, C07C 229/06, C07C 227/18

(54) **ENNIATINE UND ENNIATINDERIVATE ZUR BEKÄMPFUNG VON ENDOPARASITEN**
ENNIATINES AND ENNIATINE DERIVATES USED TO CONTROL ENDOPARASITES
ENNIATINES ET DERIVES D'ENNIATINES UTILISES DANS LA LUTTE CONTRE LES ENDOPARASITES

(30) Priorität: 11.06.1992 DE 4219157; 26.05.1993 DE 4317458
(43) Veröffentlichungstag der Anmeldung: 29.03.1995
(73) Patentinhaber: BAYER AG, 51368 Leverkusen (DE)
(72) Erfinder: BONSE, Gerhard, D-5000 Köln 80 (DE); LONDERSHAUSEN, Michael, D-4006 Erkrath 2 (DE); BISCHOFF, Erwin, D-5600 Wuppertal 1 (DE); MÜLLER, Hartwig, D-5620 Velbert 15 (DE); HARDER, Achim, D-5000 Köln 80 (DE); MENCKE, Norbert, D-5090 Leverkusen 3 (DE); KURKA, Peter, D-4010 Hilden (DE); JESCHKE, Peter, D-5090 Leverkusen (DE); SCHERKENBECK, Jürgen, D-5090 Leverkusen (DE)
(86) Internationale Anmeldenummer: EP9301436
(87) Internationale Veröffentlichungsnummer: WO9325543

(56) Entgegenhaltungen:
- EP-A- 0 382 173
- ZH. OBSHCH.KHIM. Bd. 38, Nr. 6, 1968, Seiten 1228 - 1239 MIKHALEVA, I.I. ET AL. 'Relation between structure and biological action in a series of enniatin B and its analogs'
- J. ANTIBIOTICS Bd. 45, Nr. 8, 1992, Seiten 1207 - 1215 TOMODA, H. ET AL. 'New cyclodepsipeptides, enniatins D, E and F produced by Fusarium sp. FO-1305' in der Anmeldung erw{hnt
- HELVETICA CHIMICA ACTA Bd. 46, 1963, Seiten 927 - 935 PLATTNER, P.A. ET AL. 'Synthesen in der Depsipeptid-Reihe'
- HELVETICA CHIMICA ACTA Bd. 46, 1963, Seiten 1715 - 1720 QUITT, P. ET AL. 'Synthesen in der Depsipeptidreihe' in der Anmeldung erw{hnt
- TETRAHEDRON LETTERS Bd. 2, 1971, Seiten 159 - 162 OVCHINNIKOV, Y. ET AL. 'The synthesis and some properties of Beauvericin'
- AGRIC.BIOL.CHEM. Bd. 43, Nr. 5, 1979, Seiten 1079 - 1083 KANAOKA, M. ET AL. 'Synthesis of Bassianolide and its two homologs, Enniatin C and Decabassianolide'
- CHEM.BER. Bd. 101, 1968, Seiten 1532 - 1539 LOSSE, G. & RAUE, H. 'Synthese von Stereoisomeren des Enniatins B'

## Beschreibung

Die vorliegende Erfindung betrifft die Verwendung von cyclischen Depsipeptiden mit 18 Ringatomen zur Bekämpfung von Endoparasiten, neue cyclische Depsipeptide mit 18 Ringatomen und Verfahren zu ihrer Herstellung.

Bestimmte cyclische Depsipeptide mit 18 Ringatomen (Enniatine) und Verfahren zu ihrer Herstellung sind bereits bekannt (vgl. z.B.: Hiroshi Tomoda et al., J. Antibiotics 45 (1992), S. 1207-1215 [Enniatine A, A₁, B, B₁, D, E und F]; P. Quitt et al., Helv. Chimica Acta 46 (1963), S. 1715-1720; P. Quitt et al., Helv. Chimica Acta 47 (1964) S. 166-173 [Enniatin A]; I.I. Mikhaleva et al., ZH. OBSHCH. KHIM Bd. 38, Nr. 6 (1968), S. 1228-1239 [Enniatin B]).

Über eine Verwendung dieser Verbindungen gegen Endoparasiten ist jedoch bisher nichts bekannt geworden (vgl. Merck Index, 10. Auflage, S. 517, Nr. 3543).

Aus EP-A 382 173 ist ein Depsipeptid mit 24 Ringatomen und seine Wirkung als Anthelmintikum bekannt geworden. Die anthelminitische Wirkung dieses Depsipeptids befriedigt jedoch nicht in jedem Fall.

Die vorliegende Erfindung betrifft:
1. Die Verwendung von cyclische Depsipeptiden mit 18 Ringatomen der allgemeinen Formel (I) 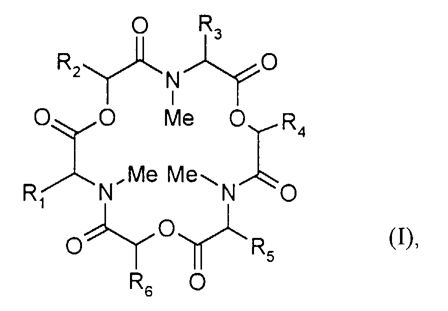 in welcher
   - R¹, R³ und R⁵: unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
   - R², R⁴ und R⁶: unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
   sowie deren optische Isomere und Racemate,
   zur Herstellung von Mitteln zur Bekämpfung von Endoparasiten in der Medizin und Tiermedizin.
2. Cyclische Depsipeptide mit 18 Ringatomen der allgemeinen Formel (Ia) 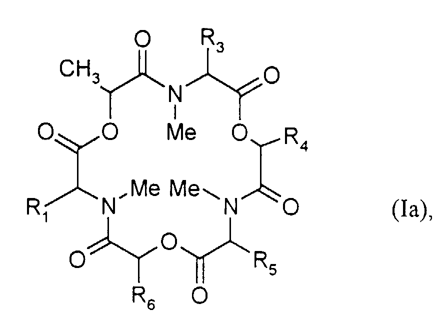 in welcher
   - R¹: für geradkettiges oder verzweigtes Alkyl mit 2 bis 8 Kohlenstoffatomen oder Cycloalkylalkyl steht,
   - R³ und R⁵: unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
   - R⁴ und R⁶: unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
   sowie deren optische Isomere und Racemate, ausgenommen die Verbindung der Formel (Ia), in welcher die Reste R¹, R³, R⁴, R⁵, R⁶ für i-Propyl stehen.
3. Verfahren zur Herstellung von cyclischen Depsipeptiden mit 18 Ringatomen der allgemeinen Formel (Ia) gemäß Punkt 2 (oben) 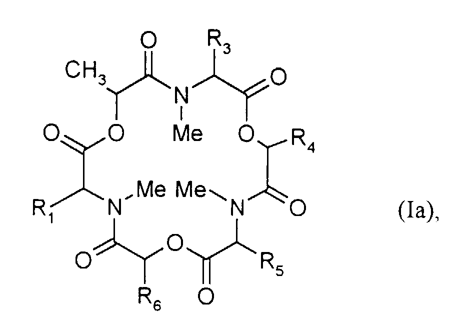 in welcher
   - R¹: für geradkettiges oder verzweigtes Alkyl mit 2 bis 8 Kohlenstoffatomen oder Cycloalkylalkyl steht,
   - R³ und R⁵: unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
   - R⁴ und R⁶: unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
   dadurch gekennzeichnet, daß man
   a) carboxy-aktivierte offenkettige Hexadepsipeptide der allgemeinen Formel (IIc) 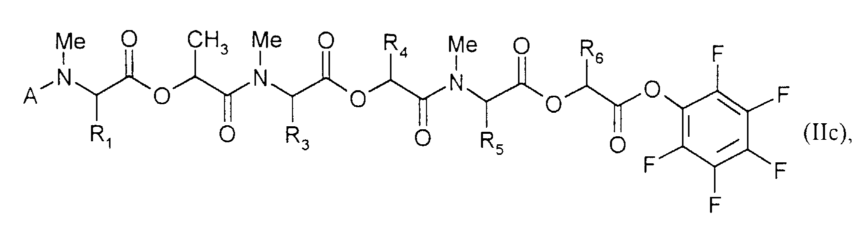 in welcher
      - A: für eine gegenüber der Aktivesterschutzgruppe selektiv entfernbare Aminoschutzgruppe, wie Benzyl oder Benzyloxycarbonyl, steht und
      - R¹, R³, R⁴, R⁵ und R⁶: die oben angegebene Bedeutung haben,
      in Gegenwart eines Hydrierkatalysators, in Gegenwart eines basischen Reaktionshilfsmittels und in Gegenwart eines Verdünnungsmittels cyclisiert, oder
   b) offenkettige Hexadepsipeptide der allgemeinen Formel (IId) 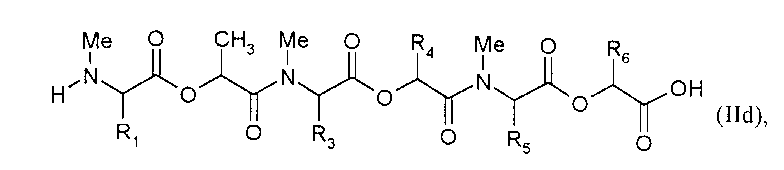 in welcher
      - R¹, R³, R⁴, R⁵ und R⁶: die oben angegebene Bedeutung haben,
      in Gegenwart eines Kupplungsreagenzes, in Gegenwart eines basischen Reaktionshilfsmittels und in Gegenwart eines Verdünnungsmittels cyclisiert.

Die offenkettigen Hexadepsipeptide der allgemeinen Formel (II) 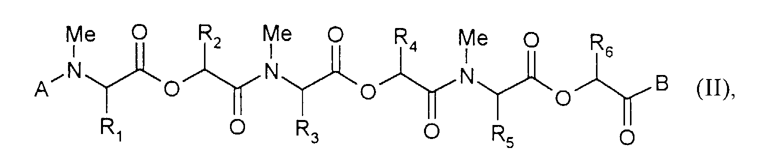 in welcher
- A: für Wasserstoff oder Benzyl steht,
oder für eine Gruppe der Formel -CO-R⁷ steht,
worin
- R⁷: geradkettiges oder verzweigtes Alkoxy, Alkenoxy oder Arylalkoxy mit bis zu 6 Kohlenstoffatomen im Alkylteil beispielsweise für tert.-Butoxycarbonyl (Boc), Benzyloxycarbonyl (Z), Ethoxycarbonyl (EtOC), Allyloxycarbonyl (AllOC), Fluorenyl-9-methoxycarbonyl (Fmoc) oder Methoxycarbonyl (MetOC) steht,
- R¹, R², R³, R⁴, R⁵ und R⁶: die unter Punkt 2 angegebene Bedeutung haben,
- B: für Hydroxyl, Halogen oder für eine dem Schutz und der gleichzeitigen Aktivierung der Carboxylgruppe dienende Aktivesterschutzgruppe, wie Pentafluorphenoxy, steht,
werden erhalten, indem man
a) Tetradepsipeptide der allgemeinen Formel (IIIb) 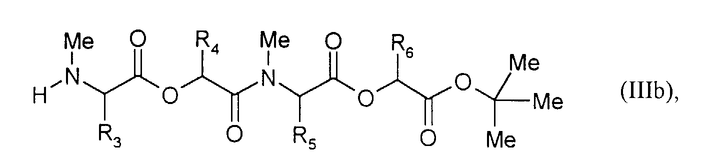 in welcher
   - R³, R⁴, R⁵ und R⁶: die oben angegebene Bedeutung haben,
   in einem ersten Reaktionsschritt mit Didepsipeptiden der allgemeinen Formel (IVb) 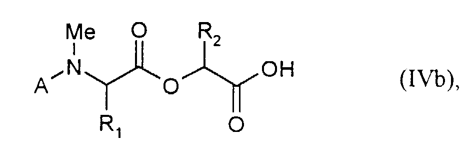 in welcher
   - A: die oben angegebene Bedeutung besitzt,
   - R¹ und R²: die oben angegebenen Bedeutung haben,
   in Gegenwart eines Kupplungsreagenzes, in Gegenwart eines basischen Reaktionshilfsmittels und in Gegenwart eines Verdünnungsmittels umsetzt,
   dann in einem zweiten Reaktionsschritt das resultierende Hexadepsipeptid der allgemeinen Formel (IIa) 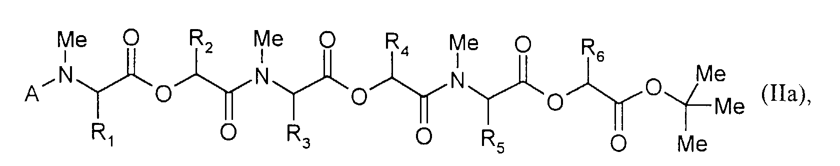 in welcher
   - A: die oben angegebene Bedeutung besitzt,
   - R¹, R², R³, R⁴, R⁵ und R⁶: die oben angegebene Bedeutung haben,
   in Gegenwart eines Verdünnungsmittels und in Gegenwart eines Protonensäure C-terminal verseift und danach die Carboxygruppe zur Aktivierung halogeniert oder in eine Aktivesterschutzgruppe, beispielsweise die Pentafluorphenylester-Gruppe, überführt oder
   in dem man
b) die z.B. gemäß Verfahren a) erhältlichen offenkettigen Hexadepsipeptide der allgemeinen Formel (IIa) 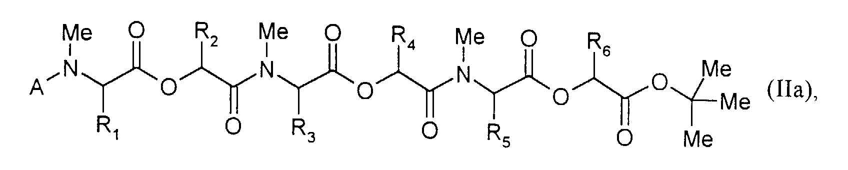 in welcher
   - A: die oben angegebene Bedeutung besitzt,
   - R¹, R², R³, R⁴, R⁵ und R⁶: die oben angegebene Bedeutung haben,
   in einem ersten Reaktionsschritt in Gegenwart eines Verdünnungsmittels und gegebenenfalls in Gegenwart einer Protonensäure C-terminal verseift,
   dann in einem zweiten Reaktionsschritt das resultierende offenkettige Hexadepsipeptid der allgemeinen Formel (IIb) 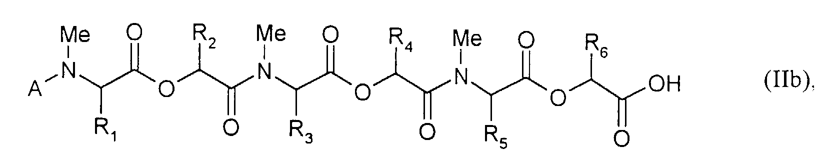 in welcher
   - A: die oben angegebene Bedeutung besitzt,
   - R¹, R², R³, R⁴, R⁵ und R⁶: die oben angegebene Bedeutung haben,
   in Gegenwart eines Verdünnungsmittels und in Gegenwart eines Katalysators N-terminal deblockiert,
   Tetradepsipeptide der allgemeinen Formel (III) 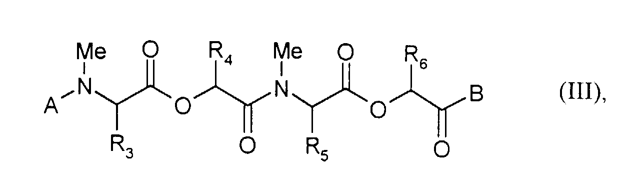 in welcher
   - A und B: die oben angegebene Bedeutung besitzen,
   - R³, R⁴, R⁵ und R⁶: die unter Punkt 2 angegebene Bedeutung haben,
   werden für den Fall, daß B für tert.-Butoxy steht, erhalten, indem man Didepsipeptide der allgemeinen Formel (Vb) 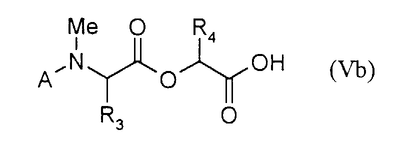 in welcher
   - A, R³ und R⁴: die oben angegebene Bedeutung haben,
   in einem ersten Reaktionsschritt mit Didepsipeptiden der allgemeinen Formel (VIb) 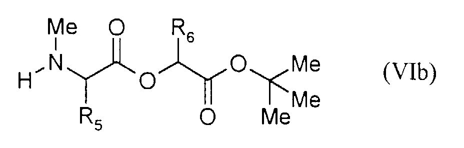 in welcher
   - R⁵ und R⁶: die oben angegebene Bedeutung haben,
   in Gegenwart eines Kupplungsreagenzes, in Gegenwart eines basischen Reaktionshilfsmittels und in Gegenwart eines Verdünnungsmittels umsetzt,
   dann in einem zweiten Reaktionsschritt das resultierende Tetradepsipeptid der allgemeinen Formel (IIIa) 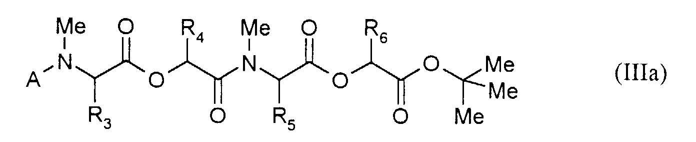 in welcher
   - A, R³, R⁴, R⁵ und R⁶: die oben angegebene Bedeutung haben,
   in Gegenwart eines Verdünnungsmittels und in Gegenwart eines geeigneten Katalysators N-terminal deblockiert.
   Didepsipeptide der allgemeinen Formel (IV) 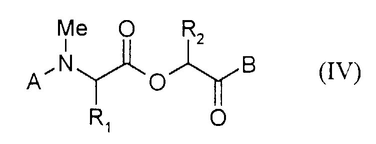 in welcher
   - A und B: die oben angegebene Bedeutung besitzen und
   - R¹ und R²: die unter Punkt 2 angegebene Bedeutung haben,
   werden für den Fall, daß B für Hydroxyl steht, erhalten, indem man N-Methyl-aminosäuren der allgemeinen Formel (VII) 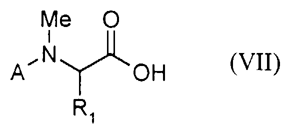 in welcher
   - A und R¹: die oben angegebene Bedeutung besitzen,
   in einem ersten Reaktionsschritt mit Alkalimetallsalzen der Formel (VIII)

   M⁺X⁻ (VIII)

   in welcher
   - M: für ein einwertiges Alkalimetallkation, vorzugsweise Lithium, Natrium, Kalium oder Cäsium, insbesondere Cäsium und
   - X: für ein Halogenid- oder Carbonatanion, vorzugsweise Carbonatanion steht,
   dann in einem zweiten Reaktionsschritt das resultierende Alkalisalz der Formel (VIIa) 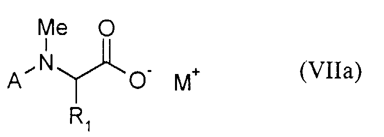 in welcher
   - A und R¹: die oben angegebene Bedeutung besitzen,
   - M: für ein salzartig gebundenes Metallkationenäquivalent steht,
   mit 2-Halogen-carbonsäurederivaten als Alkylierungsmittel der allgemeinen Formel (IX) 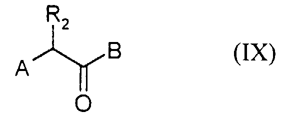 in welcher
   - R² und B: die oben angegebene Bedeutung haben und
   - Hal: für Halogen, vorzugsweise Chlor, Brom oder Iod, insbesondere Brom oder Chlor steht,
in Gegenwart eines Verdünnungsmittels umsetzt,
danach in einem dritten Reaktionsschritt, für den Fall, daß B für tert.-Butoxy steht,
das resultierende Didepsipeptid der allgemeinen Formel (IVa) 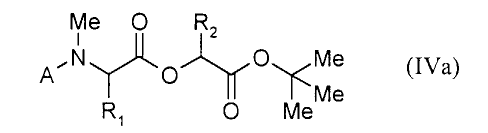 in welcher
- A, R¹ und R²: die oben angegebene Bedeutung besitzen,
in Gegenwart eines Verdünnungsmittels und gegebenenfalls in Gegenwart einer Protonensäure C-terminal verseift.

Didepsipeptide der allgemeinen Formel (V) 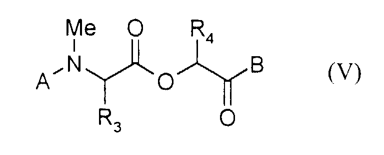 in welcher
- A und B: die oben angegebene Bedeutung besitzen,
- R³ und R⁴: die unter Punkt 2 angegebene Bedeutung haben,
werden für den Fall, daß B für Hydroxyl steht, erhalten, indem man N-Methyl-aminosäuren der allgemeinen Formel (X) 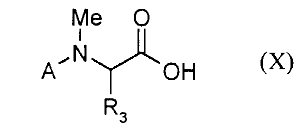 in welcher
- A und R³: die oben angegebene Bedeutung besitzen,
in einem ersten Reaktionsschritt mit Alkalimetallsalzen der Formel (VIII)

M⁺X⁻ (VIII)

in welcher
- M und X: die oben angegebene Bedeutung haben,
dann in einem zweiten Reaktionsschritt das resultierende Alkalisalz der Formel (Xa) 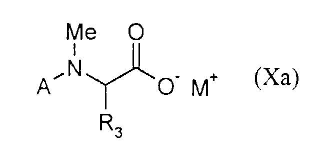 in welcher
- A, R³ und M: die oben angegebene Bedeutung haben,
mit 2-Halogen-carbonsäurederivaten als Alkylierungsmittel der allgemeinen Formel (XI) 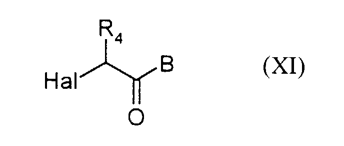 in welcher
- B, R² und Hal: die oben angegebene Bedeutung haben,
in Gegenwart eines Verdünnungsmittels umsetzt,
danach in einem dritten Reaktionsschritt, für den Fall, daß B für tert.-Butoxy steht, das resultierende Didepsipeptid der allgemeinen Formel (Va) 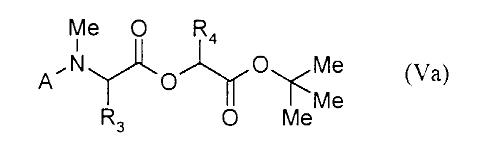 in welcher
- A, R³ und R⁴: die oben angegebene Bedeutung besitzen,
in Gegenwart eines Verdünnungsmittels und gegebenenfalls in Gegenwart einer Protonensäure C-terminal verseift.
Didepsipeptide der allgemeinen Formel (VI) 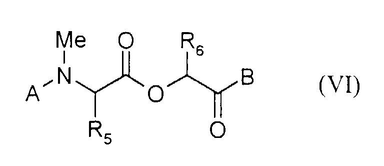 in welcher
- A und B: die oben angegebene Bedeutung besitzen,
- R⁵ und R⁶: die unter Punkt 2 angegebene Bedeutung haben,
werden für den Fall, daß B für Wasserstoff steht, erhalten, indem man N-Methyl-aminosäuren der allgemeinen Formel (XII) 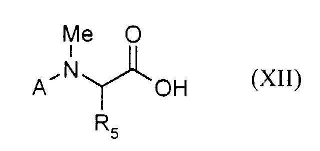 in welcher
- A und R⁵: die oben angegebene Bedeutung besitzen,
in einem ersten Reaktionsschritt mit Alkalimetallsalzen der Formel (VIII)

M⁺X⁻ (VIII)

in welcher
- M und X: die oben angegebene Bedeutung haben,
dann in einem zweiten Reaktionsschritt das resultierende Alkalisalz der Formel (XIIa) 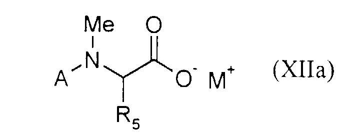 in welcher
- A, R⁵ und M: die oben angegebene Bedeutung haben,
mit 2-Halogen-carbonsäurederivaten als geeignete Alkylierungsmittel der allgemeinen Formel (XIII) 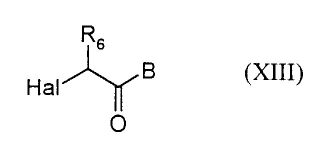 in welcher
- B, R⁶ und Hal: die oben angegebene Bedeutung haben,
in Gegenwart eines Verdünnungsmittels umsetzt,
danach in einem dritten Reaktionsschritt für den Fall, daß B für tert.-Butoxy steht, das resultierende Didepsipeptid der allgemeinen Formel (VIa) 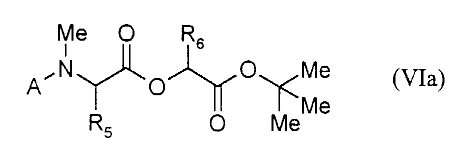 in welcher
- A, R⁵ und R⁶: die oben angegebene Bedeutung haben,
in Gegenwart eines Katalysators und in Gegenwart eines Verdünnungsmittels N-terminal deblockiert.

Die Verbindungen der allgemeinen Formel (I) eignen sich hervorragend zur Bekämpfung von Endoparasiten, besonders auf dem Gebiet der Veterinärmedizin.

Die erfindungsgemäßen cyclischen Depsipeptide mit 18 Ringatomen (Enniatine) sind durch die Formel (I) allgemein definiert.

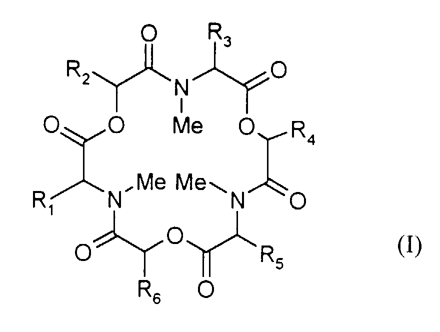

Bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl, das gegebenenfalls durch Reste aus der Reihe Halogen, insbesondere Fluor, Chlor, Brom oder Iod, Hydroxy, C₁-C₄-Alkoxy, insbesondere Methoxy oder Ethoxy, C₁-C₄-Alkyl, insbesondere Methyl, substituiert sein kann, stehen;
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl, das gegebenenfalls durch Reste aus der Reihe Halogen, insbesondere Fluor, Chlor, Brom oder Iod, Hydroxy, C₁-C₄-Alkoxy, insbesondere Methoxy oder Ethoxy, C₁-C₄-Alkyl, insbesondere Methyl, substituiert sein kann, stehen;
sowie deren optische Isomere und Racemate.

Besonders bevorzugt sind Verbindungen der Formel (I)
in welcher
- R¹, R³ und R⁵: unabhängig voneinander für Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Isoheptyl, sec.-Heptyl, Oktyl, Isooktyl, sec.-Oktyl, Cyclopentylmethyl, Cyclohexylmethyl, Cycloheptylmethyl, Phenylmethyl, das gegebenenfalls durch einen oder mehre gleiche oder verschiedene der oben angegebenen Reste substituiert sein kann, stehen;
- R², R⁴ und R⁶: unabhängig voneinander für Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Cyclopentylmethyl, Cyclohexylmethyl, Cycloheptylmethyl, Phenyl, Phenylmethyl, das gegebenenfalls durch einen oder mehre gleiche oder verschiedene der oben angegebenen Reste substituiert sein kann, stehen;
sowie deren optische Isomere und Racemate.

Ganz besonders bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Cyclohexylmethyl, Phenylmethyl, stehen;
- R², R⁴ und R⁶: unabhängig voneinander für Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Cyclohexylmethyl, Phenylmethyl, das gegebenenfalls durch einen oder mehrere gleiche oder verschiedene der oben angegebenen Reste substituiert sein kann, stehen;
sowie deren optische Isomere und Racemate.

Im Sinne der vorliegenden Erfindung können alle Verbindungen der allgemeinen Formel (I), die in optisch aktiven, stereoisomeren Formen oder als racemische Gemische vorliegen könne, verwendet werden. Vorzugsweise werden jedoch die optisch aktiven, stereoisomeren Formen der Verbindungen der allgemeinen Formel (I) erfindungsgemäß verwendet.

Im einzelenen seiein folgende Verbindungen der allgemeinen Formel (I) genannt, in welcher
die Reste R¹ bis R⁶ die folgende Bedeutung haben:

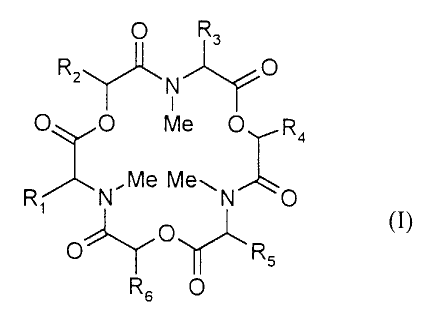

| R¹ | R² | R³ | R⁴ | R⁵ | R⁶ |
|---|---|---|---|---|---|
| -CHMECH₂Me | -CH₂Phe | -CHMECH₂Me | -Me | -CHMeCH₂Me | -Me |
| -CHMECH₂Me | -CH₂Phe | -CHMECH₂Me | -Me | -CHMeCH₂Me | -CH₂-Phe |
| -CHMECH₂Me | -(CH₂)₃-Me | -CHMECH₂Me | -Me | -CHMeCH₂Me | -Me |
| -CHMECH₂Me | -(CH₂)₃-Me | -CHMECH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CHMeCH₂Me | -Me | -CH₂Me | -Me |
| -CHMeCH₂Me | -Me | -CHMeCH₂Me | -Me | -(CH₂)₂-Me | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -CHMe₂ | -CHMe₂ | -CHMe₂ | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CH₂Me | -CHMe₂ | -CH₂-Me | -Me |
| (CH₂)₂-Me | -CHMe₂ | (CH₂)₂-Me | -Me | (CH₂)₂-Me | -Me |
| (CH₂)₂-Me | -CHMe₂ | (CH₂)₂-Me | -CHMe₂ | (CH₂)₂-Me | -Me |
| (CH₂)₃-Me | -CHMe₂ | (CH₂)₃-Me | -Me | (CH₂)₃-Me | -Me |
| (CH₂)₃-Me | -CHMe₂ | (CH₂)₃-Me | -CHMe₂ | (CH₂)₃-Me | -Me |
| Me = Methyl; Phe = Phenyl | | | | | |

Von den neuen Verbindungen der allgemeinen Formel (Ia) sind diejenigen bevorzugt und besonders bevorzugt in denen die Substituenten die oben angegebenen bevorzugten Definitionen besitzen.

Die Verbindungen der allgemeinen Formel (I) sind teilweise bekannt (durch Isolierung, vgl. z.B.: R. Zocher et al., J. Antibiotics 45 (1992) S. 1273-1277 [Enniatine A, B und C]; Hiroshi Tomoda et al. J. Antibiotics 45 (1992) S. 1207-1215 [Enniatine A, A₁, B, B₁, D, E und F]; durch Synthese, vgl. z.B.: P. Quitt et al., Helv. Chimica Acta 46 (1963) S. 1715-1720; P. Quitt et al., Helv. Chimica Acta 47 (1964) S. 166-173 [Enniatin A]; Pl. A. Plattner et al., Helv. Chimica Acta 46 (1963) S. 927-935 [Enniatin B]; Yu. A. Ovchinnikov et al. Tetrahedron Lett.2 (1971) S. 159-162; R. W. Roeske et al. Biochem. Biophys. Res. Commun. 57 (1974) S. 554-561 [Beauvericin]; Yu. A. Ovchinnikov et al., Zh. Obshch. Khim. 42 (10) (1972) S. 2320-2334; ref. C.A. 78, 58 77 k) oder können nach den dort angegebenen Verfahren erhalten werden.

Es wurde überraschenderweise gefunden, daß auch die erfindungsgemäßen Verbindungen der Formel (I) nach dem von U. Schmidt et al. für makrocyclische Peptidalkaloide angewendeten Verfahren (vgl. z.B. U. Schmidt et al. in Synthesis (1991)) S, 294-300 [Didemnin A, B und C]; Angew, Chem. 96 (1984) S. 723-724 [Dolastatin 3]; Angew. Chem. 102 (1990) S. 562-563 [Fenestin A]; Angew. Chem. 97 (1985) S. 606-607 [Ulicyclamid]; J. Org. Chem. 47 (1982) S. 3261-3264) dargestellt werden können.

Die Verbindungen der allgemeinen Formel (I) lassen sich nach den oben unter Punkt 3 angegebenen Verfahren a) und b) herstellen.

Setzt man bei Verfahren 3a zur Herstellung der neuen cyclischen Hexadepsipeptide (Enniatine) (I) als Verbindungen der Formel (IIc) N-Benzyloxycarbonyl-N-methyl-L-isoleucyl-D-lactyl-N-methyl-L-isoleucyl-D-lactyl-N-methyl-L-isoleucyl-D-milchsäurepentafluorphenylester ein, so läßt sich das Verfahren durch folgendes Reaktionsschema wiedergeben:

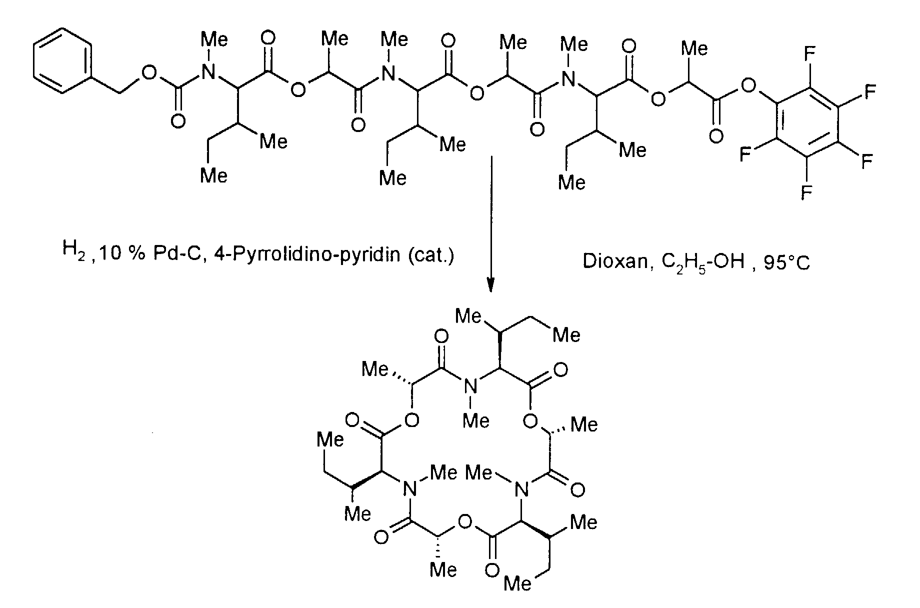

Die zur Durchführung des erfindungsgemäßen Verfahrens 3a als Ausgangsstoffe benötigten carboxy-aktivierten Derivate der offenkettigen Hexadepsipeptide sind durch die Formel (IIc) allgemein definiert. In dieser Formel stehen A und R¹ bis R⁶ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten carboxy-aktivierten Pentafluorphenylester der Formel (IIc) sind neu. Sie können nach literaturbekannten Verfahren erhalten werden (vgl. L. Kisfaludy et al. J. Org. Chem 35 (1970), S. 3563; L. Kisfaludy et al. J. Org. Chem 44 (1979), S. 654-655)). Ihre Herstellung wird weiter unten beschrieben.

Im einzelnen seien folgende Verbindungen der allgemeinen Formel (IIC) genannt, in welcher die Reste A und R¹ die folgende Bedeutung haben:

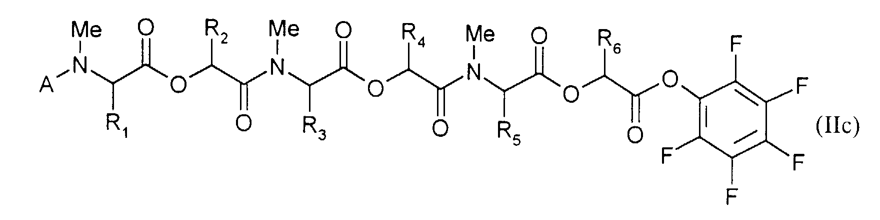

| A | R¹ | R² | R³ | R⁴ | R⁵ | R⁶ |
|---|---|---|---|---|---|---|
| Bn | -CHMECH₂Me | -CH₂Phe | -CHMECH₂Me | -Me | -CHMeCH₂Me | -Me |
| Bn | -CHMECH₂Me | -CH₂Phe | -CHMECH₂Me | -Me | -CHMeCH2Me | -CH₂-Phe |
| Z | -CHMECH₂Me | -(CH₂)₃-Me | -CHMECH₂Me | -Me | -CHMeCH₂Me | -Me |
| Z | -CHMECH₂Me | -(CH₂)₃-Me | -CHMECH₂Me | -Me | -CHMeCH₂Me | -Me |
| Bn | -CHMECH₂Me | -(CH₂)₃-Me | -CHMeCH₂Me | -Me | -CHMeCH₂Me | -(CH₂)₃-Me |
| Bn | -CHMe₂ | -CH₂-Phe | -CHMeCH₂Me | -Me | -CHMeCH₂Me | -Me |
| Z | -CH₂-Phe | -CHMe₂ | -CH₂-Phe | -CHMe₂ | -CHMeCH₂Me | -CHMe₂ |
| Bn | -CH₂CHMe₂ | -CH₂-Phe | -CH₂CHMe₂ | -Me | -CH₂CHMe₂ | -CH₂-Phe |
| Bn | -CH₂-Me | -Me | -CH₂-Me | -Me | -CH₂-Me | -Me |
| Z | -(CH₂)₂-Me | -Me | -(CH₂)₂-Me | -Me | -(CH₂)₂-Me | -Me |
| Bn | -(CH₂)₃-Me | -Me | -(CH₂)₃-Me | -Me | -(CH₂)₃-Me | -Me |
| Z | -CHMeCH₂Me | -Me | -CHMeCH₂Me | -Me | -CHMeCH₂Me | -CH₂-Me |
| Z | -CHMeCH₂Me | -Me | -CHMeCH₂Me | -Me | -CHMeCH₂Me | -(CH₂)₂-Me |
| Z | -CHMeCH₂Me | -Me | -CHMeCH₂Me | -Me | -CHMeCH₂Me | -(CH₂)₃-Me |
| Z | -CHMeCH₂Me | -Me | -CHMeCH₂Me | -Me | -CH₂Me | -Me |
| Z | -CHMeCH₂Me | -Me | -CHMeCH₂Me | -Me | -(CH₂)₂-Me | -Me |
| Z | -CHMeCH₂Me | -Me | -CHMeCH₂Me | -CHMe₂ | -CHMeCH₂Me | -Me |
| Bn | -CH₂-Phe | -CHMe₂ | -CH₂-Phe | -Me | -CH₂-Phe | -Me |
| Z | -CHMe₂ | -CHMe₂ | -CHMe₂ | -Me | -CHMe₂ | -Me |
| Z | -CHMe₂ | -CHMe₂ | -CHMe₂ | -CHMe₂ | -CHMe₂ | -Me |
| Bn | -CH₂-Me | -CHMe₂ | -CH₂-Me | -Me | -CH₂-Me | -Me |
| Bn | -CH₂-Me | -CHMe₂ | -CH₂-Me | -CHMe₂ | -CH₂-Me | -Me |
| Bn | (CH₂)₂-Me | -CHMe₂ | (CH₂)₂-Me | -Me | (CH₂)₂-Me | -Me |
| Bn | (CH₂)₂-Me | -CHMe₂ | (CH₂)₂-Me | -CHMe₂ | (CH₂)₂-Me | -Me |
| Bn | (CH₂)₃-Me | -CHMe₂ | (CH₂)₃-Me | -Me | (CH₂)₃-Me | -Me |
| Bn | (CH₂)₃-Me | -CHMe₂ | (CH₂)₃-Me | -CHMe₂ | (CH₂)₃-Me | -Me |
| Bn: -CH₂-Phenyl; Z: -CO-O-CH₂-Phenyl; Me = Methyl; Phe = Phenyl. | | | | | | |

Die Cyclisierung der Verbindungen der Formel (IIc) wird vorzugsweise in Gegenwart eines geeigneten Hydrierkatalysators und in Gegenwart eines basischen Reaktionshilfsmittel unter Verwendung von Verdünnungsmitteln durchgeführt.

Als Katalysatoren zur Durchführung des erfindungsgemäßen Verfahrens 3a kommen alle üblichen Hydrierkatalysatoren in Frage. Vorzugsweise verwendet man Edelmetallkatalysatoren, wie beispielsweise Platin, Platinoxid, Palladium oder Ruthenium gegebenenfalls auf einem geeigneten Träger wie beispielsweise Kohlenstoff oder Siliciumdioxid.

Als basische Reaktionshilfsmittel können alle geeigneten Säurebindemittel eingesetzt werden, wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Triethylamin, Trimethylamin, Tribenzylamin, Triisopropylamin, Tributylamin, Triben-zylamin, Tricyclohexylamin, Triamylamin, Trihexylamin, N,N-Dimethyl-anilin, N,N-Dimethyl-toluidin, N,N-Dimethyl-p-aminopyridin, N-Methylpyrrolidin, N-Methyl-piperidin, N-Methyl-imidazol, N-Methyl-pyrrol, N-Methylmorpholin, N-Methyl-hexamethylenimin, Pyridin, 4-Pyrrolidino-pyridin, 4-Dimethylamino-pyridin, Chinolin, α-Picolin, β-Picolin, Isochinolin, Pyrimidin, Acridin, N,N,N',N'-Tetramethylen-diamin, N,N,N',N'-Tetra-ethylendiamin, Chinoxalin, N-Propyl-diisopropylamin, B-Ethyl-diisopropylamin, N,N'-Di-methyl-cyclohexylamin, 2,6-Lutidin, 2,4-Lutidin, Triethylendiamin, Diazabicyclooctan (DABCO), Diazabicyclononen (DBN) oder Diazabicycloundecan (DBU).

Vorzugsweise verwendet man Heteroaromaten, wie beispielsweise Pyridin, N-Methylimidazol oder 4-Pyrrolidino-pyridin.

Als Verdünnungsmittel zur Durchführung des erfindungsemäßen Verfahrens 3a kommen alle inerten organischen Lösungsmittel in Frage.

Als Beispiele sind zu nenenn: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Di-n-butylether, Di-isobutylether, Diisoamylether, Diisopropylether, Anisol, Phenetol, Cyclohexylmethylether, Diethylether, Ethylenglycoldimethylether, Tetrahydrofuran, Dioxan, Dichlordiethylether; Nitrokohlenwasserstoffe wie Nitromethan, Nitroethan, Nitrobenzol, Chlornitrobenzol, o-Nitriltoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Nonan, Cymol, Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylformamid, N,N-Dimethylformamid, N-Methyl-pyrrolidon; Ketone wie Aceton, Methylethylketon. Auch Gemische der genannten Lösungs- und Verdünnungsmittel kommen in Betracht.

Bevorzugt sind Ether, wie beispielsweise Dioxan, und Gemische aus Alkoholen und Ether.

Das Verfahren 3a wird durchgeführt, indem man Verbindungen der Formel (IIc) in Gegenwart eines basischen Reaktionshilfsmittels und eines geeigneten Hydrierkatalysators in Gegenwart von Wasserstoff in einem Verdünnungsmittel unter Hochverdünnungsbedingungen erhitzt.

Die Reaktionsdauer beträgt ca. 4 bis 20 Stunden. Die Reaktion wird bei Temperaturen zwischen +20°C und +200°C, bevorzugt zwischen +70°C und +155°C durchgeführt. Vorzugsweise arbeitet man unter einer Inertgasatmosphäre und bei dem Druck, der sich beim Erhitzen auf die erforderliche Reaktionstemperatur unter den Reaktionsbedingjngen einstellt.

Zur Durchführung des erfindungsgemäßen Verfahrens 3a wird eine Dioxanlösung des N-Benzyloxycarbonyl-N-methyl-L-isoleucyl-D-lactyl-N-methyl-L-isoleucyl-D-lactyl-N-methyl-L-isoleucyl-D-milchsäure-pentafluorphenylesters der Formel (IIc) innerhalb von 2 bis 10 Stunden in die schnell gerührte Suspension äquimolarer Mengen eines geeigneten Hydrierkatalysators, beispielsweise Palladium/Kohle, in überschüssigem Dioxan unter ständigem Durchleiten von Wasserstoff bei 95°C getropft. Als Katalysatoren enthält die Lösung im allgemeinen 0,5 bis 2,5 Mol, vorzugsweise 1,0 bis 2,0 Mol, an 4-Pyrrolidino-pyridin und 0,5 bis 10 %, vorzugsweise 2 bis 5 Alkohol (auf das Lösungsmittel bezogen).

Alternativ können neben N-Benzyloxycarbonyl- auch N-Benzyl- und N-tert.-Butoxycarbonylsubstituierte Pentafluorphenylester der Formel (IIc) Verwendung finden, letztere lassen sich nach U. Schmidt (vgl. u.B.: U. Schmidt et al., Synthesis (1991) S. 294-300 [Didemnin A, B und C] in einem Zweiphasensystem cyclisieren.

Nach vollendeter Umsetzung wird das Reaktionsgemisch abgekühlt, der gesamte Reaktionsansatz im Vakuum eingeengt, mit einem organischen Lösungsmittel extrahiert und in an sich bekannter Weise aufgearbeitet. Die anfallenden Produkte lassen sich in üblicher Weise durch Umkristallisieren, Vakuumdestillation oder Säulenchromatographie reinigen (vgl. auch die Herstellungsbeispiele).

Schließlich beruht die Erfindung auf der überraschenden Feststellung, daß auch C- und N-terminal deblockierte offenkettige Hexapeptide in Gegenwart geeigneter Kupplungsreagenzien und in Gegenwart eines basischen Reaktionshilfsmittels in einem Verdünnungsmittel unter Hochverdünnungsbedingungen cyclisieren können.

Setzt man bei Verfahren 3a zur Herstellung der neuen cyclischen Hexadepsipeptide (Enniatine) (I) als Verbindungen der Formel (II) N-Methyl-L-isoleucyl-D-lactyl-N-methyl-L-isoleucyl-D-lactyl-N-methyl-L-isoleucyl-D-milchsäure ein, so läßt sich das Verfahren durch folgendes Reaktionsschema wiedergeben:

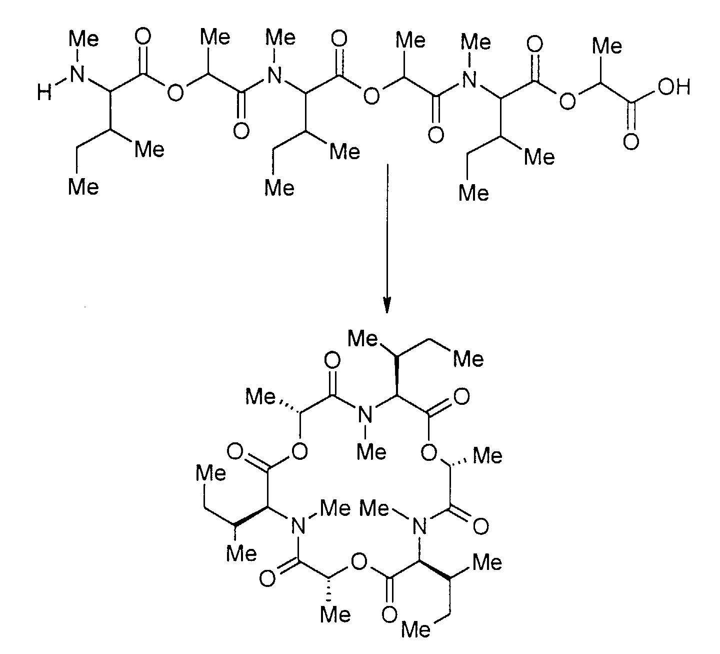

Die zur Durchführung des erfindungsgemäßen Verfahrens 3b als Ausgangsstoffe benötigten offenkettigen Hexadepsipeptide sind durch die Formel (IId) allgemein definiert. In dieser Formel stehen R¹ bis R⁶ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Hexadepsipeptide der Formel (IId) können nach den weiter unten beschriebenen Verfahren erhalten werden.

Im einzelnen seien folgende Verbindungen der allgemeinen Formel (IId) genannt, in welcher die Reste R¹ bis R⁶ die folgende Bedeutung haben:

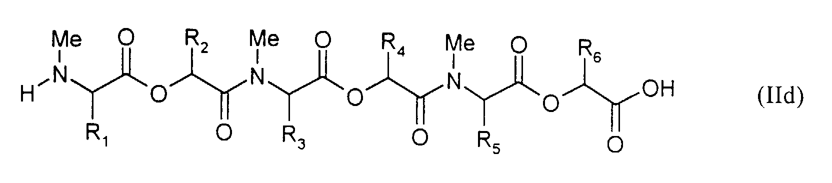

| R¹ | R² | R³ | R⁴ | R⁵ | R⁶ |
|---|---|---|---|---|---|
| -CHMECH₂Me | -CH₂Phe | -CHMECH₂Me | -Me | -CHMeCH₂Me | -Me |
| -CHMECH₂Me | -CH₂Phe | -CHMECH₂Me | -Me | -CHMeCH₂Me | -CH₂-Phe |
| -CHMECH₂Me | -(CH₂)₃-Me | -CHMECH₂Me | -Me | -CHMeCH₂Me | -Me |
| -CHMECH₂Me | -(CH₂)₃-Me | -CHMECH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CHMeCH₂Me | -Me | -CH₂Me | -Me |
| -CHMeCH₂Me | -Me | -CHMeCH₂Me | -Me | -(CH₂)₂-Me | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -CHMe₂ | -CHMe₂ | -CHMe₂ | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CH₂Me | -CHMe₂ | -CH₂-Me | -Me |
| (CH₂)₂-Me | -CHMe₂ | (CH₂)₂-Me | -Me | (CH₂)₂-Me | -Me |
| (CH₂)₂-Me | -CHMe₂ | (CH₂)₂-Me | -CHMe₂ | (CH₂)₂-Me | -Me |
| (CH₂)₃-Me | -CHMe₂ | (CH₂)₃-Me | -Me | (CH₂)₃-Me | -Me |
| (CH₂)₃-Me | -CHMe₂ | (CH₂)₃-Me | -CHMe₂ | (CH₂)₃-Me | -Me |
| Me = Methyl; Phe = Phenyl | | | | | |

Als Kupplungsreagenzien zur Durchführung des Verfahrens 3b finden alle, die zur Herstellung einer Amidbindung geeignet sind (vgl. z.B.: Houben-Weyl, Methoden der organischen Chemie, Band 15/2, Bodanszky et al.; Peptide Synthesis 2nd ed. (Wiley & Sons, New York 1976) oder Gross, Meienhofer, The Peptides; Analysis synthesis, biology (Academic Press, New York 1979), Verwendung. Vorzugsweise werden folgende Methoden herangezogen: Aktivestermethode mit Pentachlor- (Pcp) und Pentafluorphenol (Pfp), N-Hydroxysuccinimid, N-Hydroxy-5-norbornen-2,3-dicarboxamid (HONB), 1-Hydroxy-benzotriazol (HOBt) oder 3-Hydroxy-4-oxo-3,4-dihydro-1,2,3-benzotriazin als Alkoholkomponenten, Kupplung mit Carbodiimiden wie Dicyclohexylcarbodiimid (DCC) nach dem DCC-Additiv-Verfahren, oder mit n-Propanphosphonsäureanhydrid (PPA) und Gemischt-Anhydrid-Methode mit Pivaloylchlorid, Ethyl- (EEDQ) und Isobutyl-chlorformiat (IIDQ) oder Kupplung mit Phosphoniumreagenzien, wie Benzotriazol-1-yl-oxy-tris(dimethylaminophosphonium)-hexafluorophosphanat (BOP), Bis(2-oxo-3-oxazolidinyl)phosphoniumsäurechlorid (BOP-Cl), oder mit Phosphonsäureesterreagenzien, wie Cyanphosphonsäurediethylester (DEPC) und Diphenylphosphorylazid (DPPA) oder Uroniumreagenzien, wie 2-(1H-Benzotriazol-1-yl)-1,1,3,3-tetramethyluroniumtetrafluoro-borat (TBTU).

Bevorzugt ist die Kupplung mit Phosphoniumreagenzien wie Bis(2-oxo-3-oxazolidinyl)-phosphoniumsäurechlorid (BOP-Cl), Benzotriazol-1-yl-oxy-tris(dimethylamino-phosphonium)-hexafluorophosphanat (BOP) und Phosphonsäureesterreagenzien, wie Cyanphosphonsäurediethylester (DEPC) oder Diphenylphosphorylazid (DPPA).

Als basische Reaktionshilfsmittel zur Durchführung des Verfahrens 3b finden die bei Verfahren 3a genannten tertiären Amine, insbesondere Trialkylamine wie Triethylamin, N,N-Diisopropylethylamin oder N-Methyl-morpholin, Verwendung.

Als Verdünnungsmittel zur Durchführung des Verfahrens 3b finden die bei Verfahren 3a genannten halogenierten Kohlenwasserstoffe, insbesondere Chlorkohlenwasserstoffe Verwendung.

Das Verfahren 3b wird durchgeführt, indem Verbindungen der Formel (IId) in Gegenwart eines der angegebenen Kupplungsreagenzien und in Gegenwart eines basischen Reaktionshilfsmittels in einem Verdünnungsmittel unter Hochverdünnungsbedingungen zusammengegeben und gerührt werden. Die Reaktionsdauer beträgt 4 bis 72 Stunden. Die Umsetzung erfolgt bei Temperaturen zwischen -5°C und +100°C, bevorzugt zwischen -5°C und +50°C, besonders bevorzugt bei 0°C bis Raumtemperatur. Es wird unter Normaldruck gearbeitet.

Zur Durchführung des erfindungsgemäßen Verfahrens 3b setzt man pro Mol an N-Methyl-L-isoleucyl-D-lactyl-N-methyl-L-isoleucyl-D-lactyl-N-methyl-L-isoleucyl-D-milchsäure der Formel (IId) im allgemeinen 1,0 bis 3,0 Mol, vorzugsweise 1,0 bis 1,5 Mol Kupplungsreagenzien, ein.

Nach vollendeter Umsetzung wird das Reaktionslösung schwach alkalisch gewaschen, die organische Phase abgetrennt, getrocknet und im Valuum eingeengt. Die anfallenden Produkte lassen sich in üblicher Weise durch Umkristallisieren, Vakuumdestillation oder Säulenchromatographie reinigen (vgl. auch die Herstellungsbeispiele).

Mit den erfindungsgemäßen Verfahren 3a und 3b sind, aus offenkettigen Hexadepsipeptiden mit sowohl L- als auch D-Konfiguration aufgebauter Depsipeptidsequenz, Cyclohexadepsipeptide (Enniatine) unter Beibehaltung der ursprünglichen Konfiguration der Ausgangsstoffe erhältlich.

Die als Ausgangsverbindungen verwendeten offenkettigen Depsipeptide können nach an sich bekannten Verfahren hergestellt werden, beispielsweise demjenigen, wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B.F. Gisin (Helv. Chim. Acta 56 (1973) S. 1476) beschrieben ist.

Die als Ausgangsmaterialien verwendeten N-Methyl-aminosäuren und 2-Halogen-carbon-säurederivate sind teilweise bekannt (vgl. z.B.: N-Methyl-aminosäuren; R. Bowmann et al. J. Chem. Soc. (1950) S. 1346; J.R. McDermott et al. Can. J. Chem. 51 (1973) S. 1915; H. Wurziger et al.; Kontakte (Merck, Darnstadt) 3 (1987) S. 8; 2-Halogencarbonsäurederivate; S.M. Birnbaum et al. Amer. Chem. Soc. 76 (1954) S. 6054, C.S. Rondestvedt, Jr. et al. Org. Reactions 11 (1960) S. 189 [Review] bzw. können nach den dort beschriebenen Verfahren erhalten werden.

Für die Kupplungsreaktion zur Darstellung der als Ausgangsverbindungen eingesetzten, offenkettigen Depsipeptide (II), (III), (IV), (V) und (VI) finden die bei Verfahren 3b genannten Kupplungsreagenzien Verwendung.

Die offenkettigen Hexadepsipeptide (II) können somit nach einem Verfahren erhalten werden, das die folgenden aufeinander folgenden Stufen umfaßt:

### a) Synthese der Didepsipeptide der Formeln (IV) bis (VI):

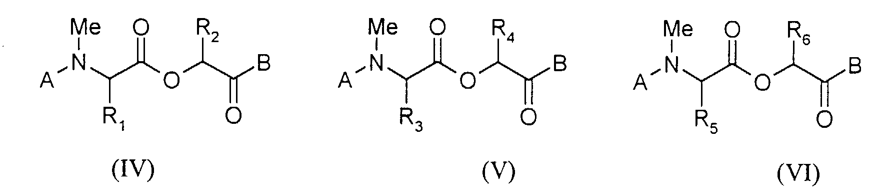

worin A eine N-terminale Schutzgruppe, wie z.B. die Benzyl oder Benzyloxycarbonylgruppe und B eine C-terminale Schutzgruppe, wie z.B. die tert.-Butoxygruppe, darstellt.

Dies entspricht für Formel (VI) dem folgenden Reaktionsschema:

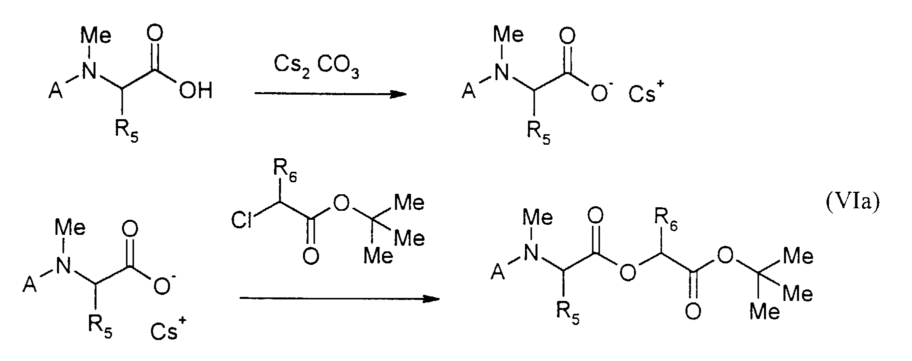

Die Herstellung der erfindungsgemäßen, enantiomeren Verbindungen der Formeln (IV), (V) und (VI) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.

Am Ende dieser Stufe kann entweder eine Entfernung der N-terminalen Schutzgruppe aus den Derivaten der Formel (VIa) in an sich bekannter Weise durchgeführt werden, beispielsweise durch katalytische Hydrierung, zur Herstellung der Derivate der Formel (VIb) oder es kann in an sich bekannter Weise eine Abspaltung der C-terminalen Schutzgruppe aus den Derivaten der Formel (IV) und (V), vorzugsweise durch Acidolyse, zur Darstellung der Derivate (IVb) und (Vb) erfolgen:

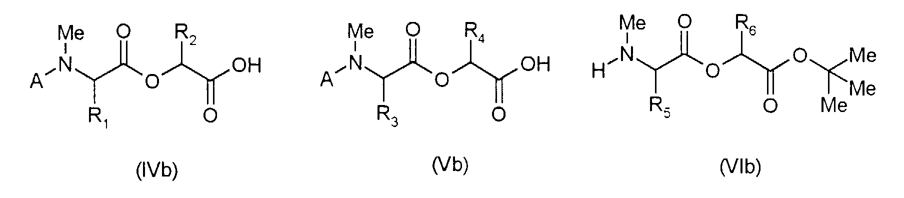

### b) Synthese der Tetradepsipeptide der Formel

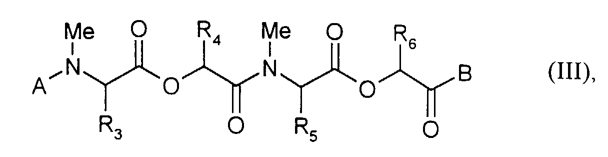

nach der folgenden Reaktionsgleichung:

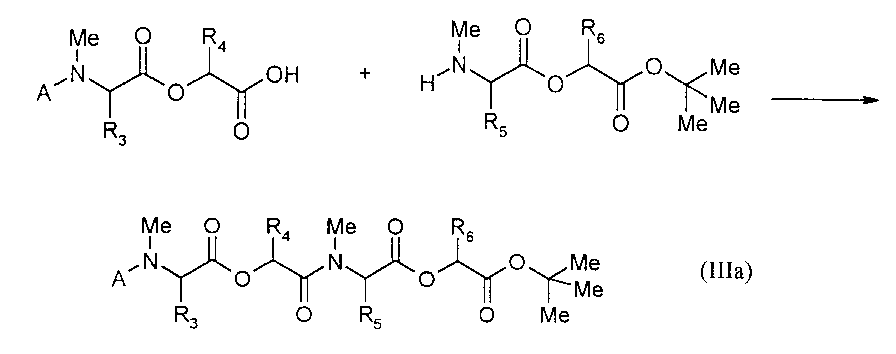

Man kann anschließend eine Entfernung der N-terminalen Schutzgruppe aus den Derivaten der Formel (IIIa) durchführen, beispielsweise durch katalytische Hydrierung wie oben angegeben, zur Herstellung der Derivate der Formel

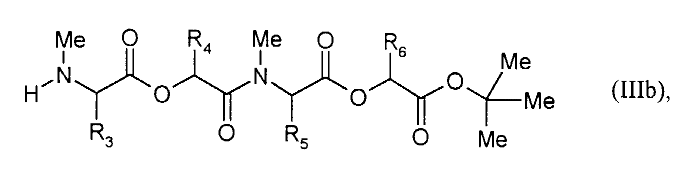

### c) Synthese der offenkettigen Hexadepsipeptide der Formel

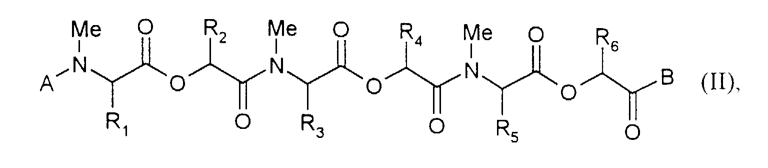

nach der folgenden Reaktionsgleichung:

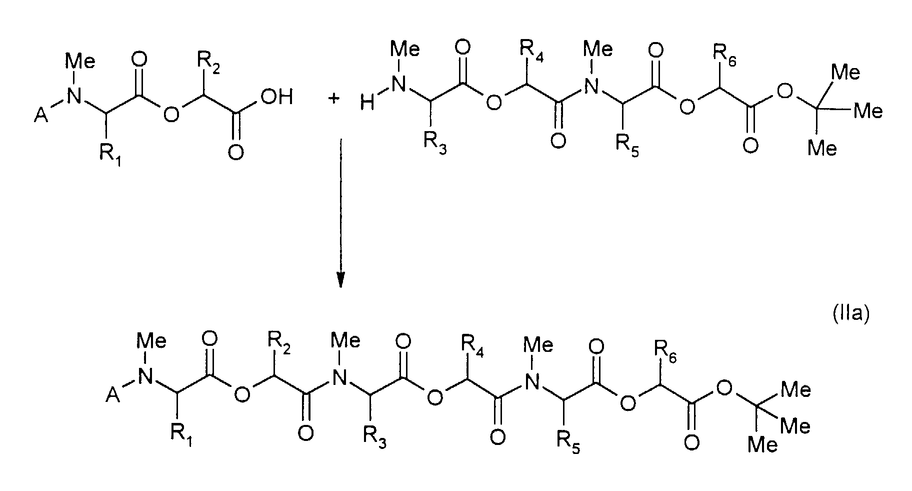

Man kann anschließend eine Entfernung der C-terminalen Schutzgruppe aus den Derivaten der Formel (IIa) in an sich bekannter Weise durchführen, beispielsweise durch Acidolyse wie oben angegeben, zur Herstellung der Derivate der Formel:

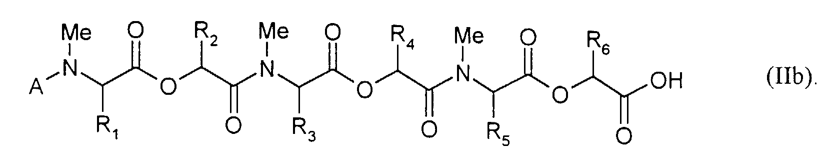

Am Ende dieser Stufe kann die Darstellung der carboxy-aktivierten Derivate der offenkettigen Hexadepsipeptide, beispielsweise der Pentafluorphenylester der Formel 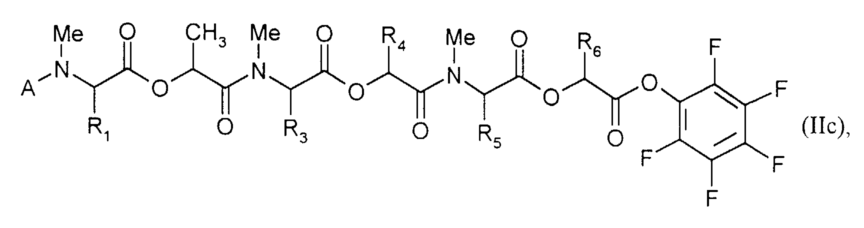 erfolgen oder es wird eine Entfernung der N-terminalen Schutzgruppe aus den Derivaten der Formel (IIb) in an sich bekannter Weise, durchgeführt, beispielsweise
durch katalytische Hydrierung wie oben angegeben, zur Herstellung der Derivate der Formel

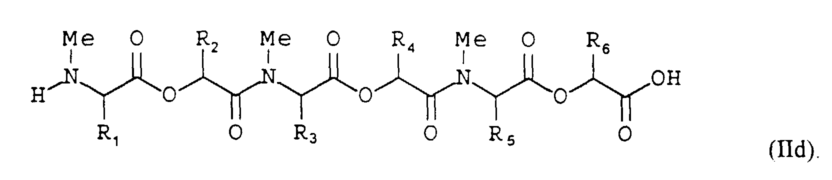

Die anfallenden Produkte lassen sich in üblicher Weise durch Umkristallisieren, Vakuumdestillation oder Säulenchromatographie reinigen (vgl. auch die Herstellungsbeispiele).

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp. Toxascaris spp., Toxocara spp., Parascaris, spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: PHysiologisch verträgliche Lösungsmittel wie Wasser, Alkohole wie Ethanol, Butanol, Benzylalkohol Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes

Konservierungsmittel sind: Benzylalkohol, Trichlorbutanol, p-Hydroxybenzoesäureester, n-Butanol

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz;

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside ganannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden enthalten den Wirkstoff in Konzentrationen von 0,5 - 90 Gew.-%, bevorzugt von 5 - 50 Gew -%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Formulierungsbeispiele

### Beispiel 1

200 g Wasser für Injektionszwecke werden 10 Minuten im Stickstoff begast. An-schließend werden in dieser Wassermenge 0,3 g Ascorbinsäure, 43,2 g Glucose gelöst und 3,55 g 0,5 M-Argininlösung zur pH-Einstellung hinzugegeben. In diesem Medium werden 0,15 g Enniatin A und 24 g hochreines Ei-Phospholipid (z.B. Pipoid E PC) dispergiert und mit stickstoffbegastem Wasser auf 300 g aufgefüllt (Wirkstoff: Phospholipid-Verhältnis = 1:160).

Diese Dispersion wird 30 Minuten bei 75°C und unter Stickstoffschutz mit einem schnellaufenden Rührwerk (z.B. Ultra-Turrax) vorhomogenisiert. Die Vordispersion wird durch einen 5 µm-Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt beträgt 100 % des Sollgehalts.

Die Dispersion wird zu 15,3 ml in 250- oder 50 ml-Flaschen abgefüllt und mit Hilfe des spin- oder shell freezing bei -65°C (Trockeneis-Ethanol-Kühlmischung) zu einem dünnen Produktkuchen eingefroren.

Das eingefrorene Produkt wird auf die auf -50°C vorgekühlten Stellflächen eines Lyophilisators gebracht und bei -30°C und 0,05 mbar 14 Stunden lang getrocknet. Die Nachtrocknung dauert 7 Stunden bei 30°C und 0,001 mbar.Das Lyophilisator wird mit Glucoselösung oder mit Wasser rekonstituiert. Der Gehalt beträgt 100 % des Sollgehalts.

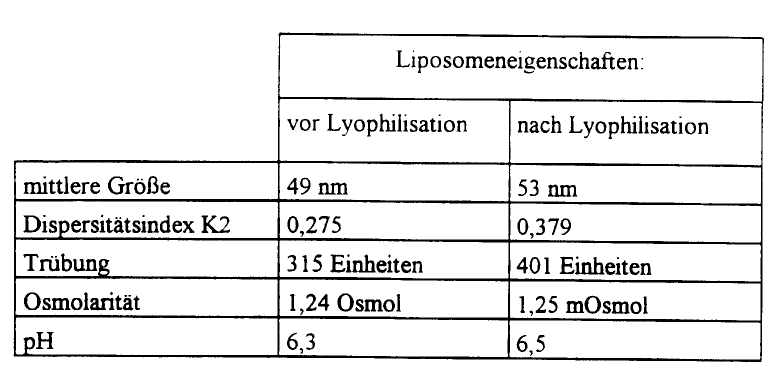

### Beispiel 2

200g Wasser für Injektionszwecke werden 10 Minuten mit Stickstoff begast. Anschließend werden in dieser Wassermenge 0,3 g Ascorbinsäure, 43,2 g Glucose gelöst und 3,55 g 0,5 M-Argininlösung zur pH-Einstellung hinzugegeben. In diesem Medium werden 0,15 g Enniatin A und 30 g hochreines Soja-Phospholipid (z.B. Phospholipon 90) dispergiert und mit stickstoffbegastem Wasser auf 300 g aufgefüllt (Wirkstoff: Phospholipid-Verhältnis = 1:200).

Diese Dispersion wird 30 Minuten bei 75°C und unter Stickstoffschutz mit einem schnellaufenden Rührwerk (z.B. Ultra-Turrax) vorhomogenisiert. Die Vordispersion wird durch einen 5 µm-Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt beträgt 100 % des Sollgehalts.

Im Gegensatz zu Beispiel 1 wird nach Abfüllen in entsprechende Flaschen ohne Einsatz des spin- oder shell freezing die Dispersion bei -65°C eingefroren.

Das Produkt wird bei +30°C und 0,05 mbar 7 h getrocknet (Haupttrocknung) und bei +30°C und 0,001 mbar nachgetrocknet.

Die Liposomeneigenschaften aus Beispiel 1 bleiben erhalten.

### Beispiel 3

200g Wasser für Injektionszwecke werden 10 Minuten mit Stickstoff begast. Anschließend werden in dieser Wassermenge 0,3 g Ascorbinsäure, 43,2 g Glucose gelöst und 3,55 g 0,5 M-Argininlösung zur pH-Einstellung hinzugegeben. In diesem Medium werden 0,24 g Enniatin A und 22,6 g hochreines, gesättigtes Phospholipid (z.B. Epikuron 200 SH) und 2,4g synthetisches DMPG-Na dispergiert und mit stickstoffbegastem Wasser auf 300 g aufgefüllt (Wirkstoff: Phospholipid-Verhältnis = 1:100).

Diese Dispersion wird 30 Minuten bei 75°C und unter Stickstoffschutz mit einem schnellaufenden Rührwerk (z.B. Ultra-Turrax) vorhomogenisiert. Die Vordispersion wird durch einen 5 µm Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt beträgt 100 % des Sollgehalts.

Die Dispersion wird zu 15,3 ml in 250- oder 50 ml-Flaschen abgefüllt und mit Hilfe des spin- oder shell freezing bei -65°C (Trockeneis-Ethanol-Kühlmischung) zu einem dünnen Produktkuchen eingefroren.

Das eingefrorene Produkt wird auf die auf -50°C vorgekühlten Stellflächen eines Lyophilisators gebracht und bei -30°C und 0,05 mbar 14 Stunden lang getrocknet. Die Nachtrocknung dauert 7 Stunden bei 30°C und 0,001 mbar.

Das Lyophilisat wird mit Glucoselösung oder Wasser rekonstituiert. Der Gehalt beträgt 100 % des Sollgehalts.

### Beispiel A

### Trichinella spiralis in vitro

Trichinenlarven werden aus der Muskulatur von Mäusen isoliert und in 0,9 % NaCl, supplementiert mit 20 µg/ml Sisomycin und 2 µg/ml Clotrimazole gewaschen. Die eigentliche Inkubation von ca. 20 Trichinen pro Messung erfolgt in 2 ml einer Lösung bestehend aus 10 g Bacto Casitone, 5 g Hefe (Yeast Extract), 2,5 g Glucose, 0,4 g KH₂PO₄, 0,4 g K₂HPO₄ pro 500 ml pH 7,2, enthaltend 10 µm/ml Sisomycin und 1 µm/ml Clotrimazole. 10 mg der zu testenden Substanz werden in 0,5 ml DMSO gelöst und so viel zum Inkubationsmedium zugesetzt, daß die Endkonzentration 100, 10 und 1 µm/ml beträgt. Nach 5 Tagen Inkubation bei 19°C wird der Versuch ausgewertet.

Enniatine A₁, B und B₁ sind bei einer Konzentration von 100 µm/ml wirksam.

### Beispiel B

Weibliche Heterakis spumose-Nematoden werden aus dem Colon und Caecum von Mäusen isoliert. 10 Weibchen werden in 1,5 ml Medium, das für den Trichinella spiarlis in-vitro-Versuch verwendet wird, 3 Tage lang bei 37°C inkubiert. Der Zusatz von Testsubstanzen erfolgt ebenfalls so, wie beim Trichinentest dargestellt. Zur Beurteilung der anthelmintischen Aktivität werden die Beweglichkeit und die Eiausscheidung im Vergleich zur Kontrolle herangezogen.

Enniatine A₁, B und B₁ sind bei einer Konzentration von 10 µm/ml wirksam.

### Beispiel C

### In vivo Nematodentest

### Haemonchus contortus / Schaf

Experimentell mit Haemonchus contortus infizierte Schaft wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 5 |
| 2 | 5 |
| 4 | 5 |
| 5 | 5 |

### Herstellungsbeispiele

### Beispiel 1:

### Cyclo(-N-methyl-L-isoleucyl-D-lactyl-N-methyl-L-isoleucyl-D-lactyl-N-methyl-L-isoleucyl-D-lactyl-)

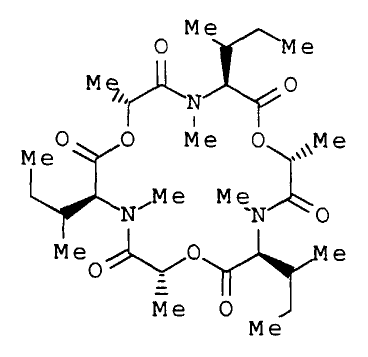

In eine schnell gerührte Suspension von 1,5 g 10%iger Palladium/Kohle in 550 ml absolutem Dioxan, das 12 ml Ethanol und 160 mg (1.08 mMol) 4-Pyrrolidino-pyridin enthält, werden bei 95 °C Innentemperatur 0.99 g (1.08 mMol) Z-(-L-MeIle-D-Lac-)₃-O-Pfp in 50 ml absolutem Dioxan im Verlauf von 6 Stunden gleichmäßig eingespritzt. Dabei leitet man Wasserstoff durch die Reaktionslösung. Anschließend werden weitere 4 Stunden bei 95 °C und 12 Stunden bei Raumtemperatur nachgerührt. Man filtriert und engt den gesamten Reaktionsansatz im Vakuum ein. Der farblose ölige Rückstand wird in Chloroform aufgenommen und zweimal mit 5 %iger Zitronensäure, zweimal mit NaHCO₃-Lösung sowie zweimal mit Wasser gewaschen. Die organische Phase wird über Natriumsulfat getrocknet und an-schließend das Lösungsmittel im Vakuum abdestilliert. Das zurückbleibende Rohprodukt kann über eine Kieselgelsäule (Kieselgel 60 - Merck, Korngröße : 0,04 bis 0,063 mm) mit dem Fließmittel Toluol:Essigsäureethylester (4:1) chromatographisch vorgereinigt werden (Reinheit 84 %). Danach erfolgt die präparative HPLC-Reinigung. Man erhält 710 mg (36,8 % der Theorie) Cyclo(-N-methvl-L-isoleucyl-D-lactyl-N-methyl-L-isoleucyl-D-lactyl-N-methyl-L-isoleucyl-D-lactyl-).
Fp.: 210-212 °C
¹H-NMR (400 MHz, CDCl₃, δ): 0,87 (t, 9H, -CH₂-CH₃; J = 7,3 Hz); 0,98; 1,44 (2d, 18H, -CH-CH₃; J = 6,5 Hz); 1,35-1,41 (br. m, 3H, -CH-CH₃); 2,02-2,04 (br. m, 6H, -CH₂-CH₃); 3,03 (s, 9H, -N-CH₃); 4,45 (m, 3H, N-CH-CO); 5,57-5,62 (m, 3H, O-CH-CO) ppm
¹³C-NMR (100 MHz, CDCl₃, δ): 10,9; 16,0; 16,6 (-CH₃); 24,8 (-CH₂-); 33,3 (-CH-); 33,9 (-N-CH₃); 61,9 (-N-CH-); 66,4 (-O-CH-); 169,3 (-CO-N-); 169,9 (-CO-O-) ppm
FAB-MS m/z (%): 598 (M⁺+H,12); 597 (37); 541 (42); 524 (14); 182 (100)

Analog können die in der nachstehenden Tabelle 1 aufgeführten Verbindungen der Formel ( I ) als LDLDLD - Stereoisomere hergestellt werden.

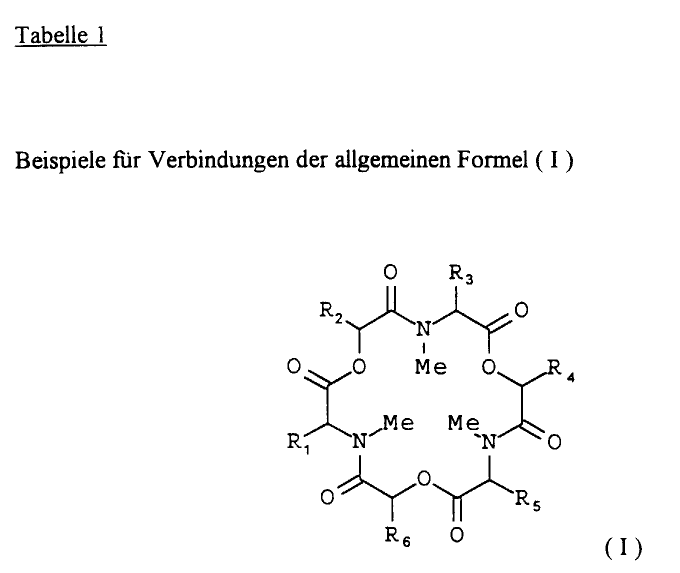

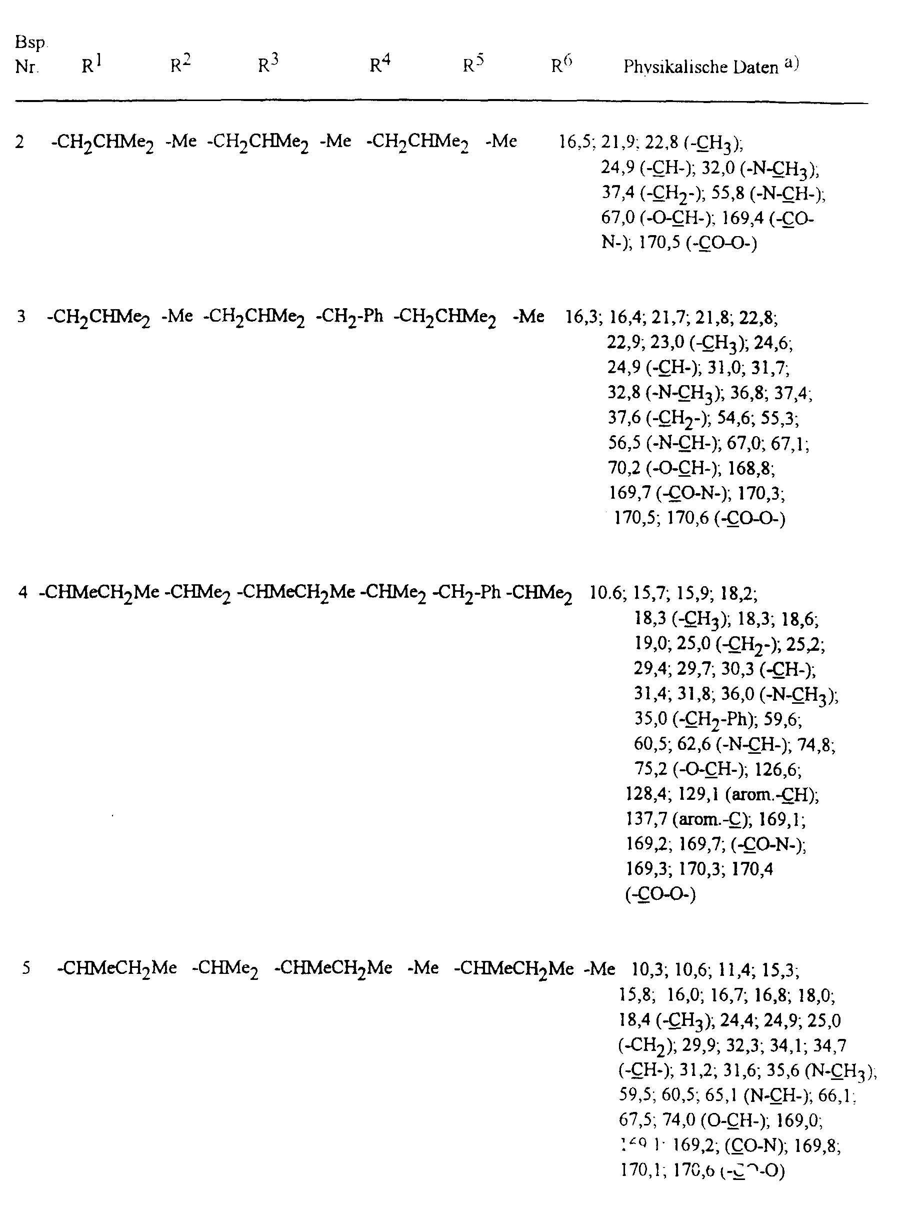

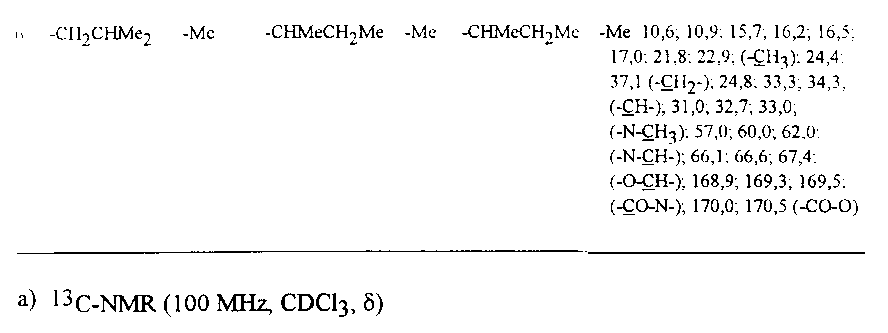

### Ausgangsstoffe der Formel ( II )

### Beispiel (II - 1)

### N-Benzyloxycarbonyl-N-methyl-L-isoleucyl-D-lactyl-N-methyl-L-isoleucyl-D-lactyl-N-methyl-L-isoleucyl-D-milchsäure-tert.-butylester

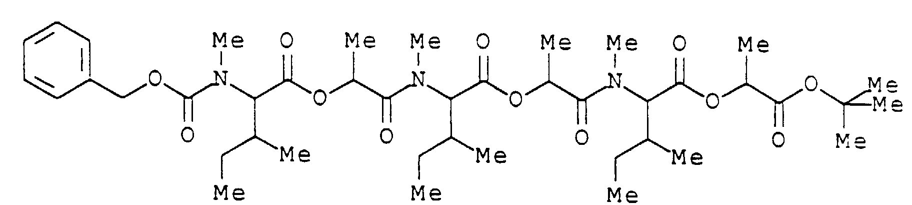

Zu einer Lösung von 5,8 g (16,5 mMol) Z-L-Melle-D-Lac-OH und 7,8 g (16,5 mMol) H-(-L-MeIle-D-Lac-)₂-O-^{t}Bu in 150 ml Methylenchlorid werden bei 0 °C 4,7 g (36,3 mMol) N,N-Diisopropylethylamin ("Hünig's Base") sowie 4,6 g (18,1 mMol) Bis(2-oxo-3-oxazolidinyl)-phosphoniumsäurechlorid (BOP-Cl) zugegeben und 4 Stunden gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Toluol : Essigsäureethylester (5:1) chromatographiert. Man erhält 10,3 g (77,4 % der Theorie) N-Benzyloxycarbonyl-N-methyl-L-isoleucyl-D-lactyl-N-methyl-L-isoleucyl-D-lactyl-N-methyl-L-isoleucyl-D-milchsäure-tert.-butylester.
FAB-MS m/z (%): 805 (M⁺,3); 749 (M⁺- H₂C=CMe₂,10); 732 (9); 793 (10); 91 (100)

### Beispiel (II - 2)

### N-Benzyloxycarbonyl-N-methyl-L-isoleucyl-D-lactyl-N-methyl-L-isoleucyl-D-lactyl-N-methyl-L-isoleucyl-D-milchsäure

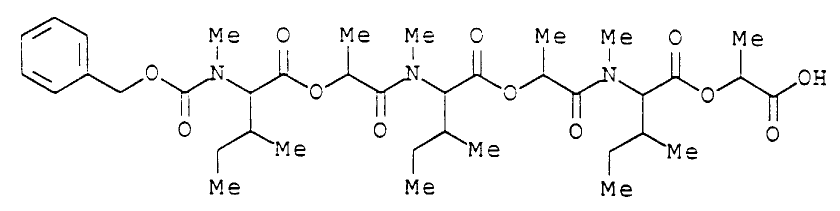

In eine auf 0 °C gekühlte Lösung von 9,2 g (11,2 mMol) Z-(-L-MeIle-D-Lac-)₃-O-^{t}Bu in 150 ml absolutem Methylenchlorid wird 20 Minuten trockenes Chlorwasserstoffgas eingeleitet. Anschließend rührt man ca. 16 Stunden bei Raumtemperatur und engt den gesamten Reaktionsansatz im Vakuum ein. Man erhält 7,1 g (82,9 % der Theorie) N-Benzyloxy-carbonyl-N-methyl-L-isoleucyl-D-lactyl-N-methyl-L-isoleucyl-D-lactyl-N-methyl-L-isoleucyl-D-milchsäure, das ohne weitere Reinigung weiter umgesetzt werden kann.
MS m/z (%): 749 (M⁺,10); 721 (1); 693 (2); 533 (0.5); 91 (100)

### Beispiel (II - 3)

### N-Benzyloxycarbonyl-N-methyl-L-isoleucyl-D-lactyl-N-methyl-L-isoleucyl-D-lactyl-N-methyl-L-isoleucyl-D-milchsäure-pentafluorphenylester

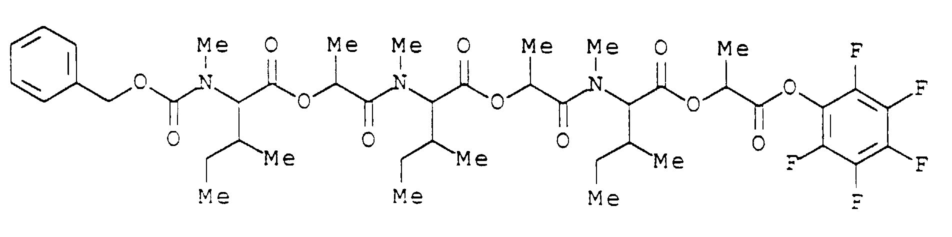

5,0g (6,67 mMol) Z-(L-Melle-D-Lac)₃-OH werden zusammen mit 1,23 g (6,67 mMol) Pentafluorphenol in 125 ml absolutem Essigsäureethylester gelöst (Inertgasatmosphäre). Dazu gibt man bei 0 °C 1,38g (6,67 mMol) Dicyclohexylcarbodiimid (DCC) und rührt noch weitere 4 Stunden bei dieser Temperatur. Nach Filtration vom ausgefallenen Dicyclohexylharnstoff wird das Filtrat im Vakuum zur Trockene eingeengt und der Rückstand über eine vorgetrocknete Kieselgelsäule (Kieselgel 60 - Merck, Korngröße : 0,04 bis 0,063 mm) mit dem Fließmittel Toluol : Essigsäureethylester (10:1) chromatographiert.

Man erhält 3,3 g (54 % der Theorie) N-Benzyloxycarbonyl-N-methyl-L-isoleucyl-D-lactyl-N-methyl-L-isoleucyl-D-lactyl-N-methyl-L-isoleucyl-D-milchsäurepentafluorphenylester als farbloses Öl.
FAB-MS m/z (%): 915 (M⁺,2); 914 (M⁺-H,4); 859 (9); 814 (1); 780 (5); 91 (100)

### Beispiel (II-4)

### N-Methyl-L-isoleucyl-D-lactyl-N-methyl-L-isoleucyl-D-lactyl-N-methyl-L- isoleucyl-D-milchsäure

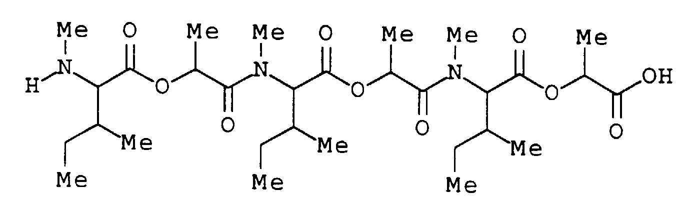

1,0 g (1,33 mMol) Z-(-L-MeIle-D-Lac-)₃-OH werden in 20 ml Ethanol in Gegenwart von 0,15 g Pd(OH)₂/Kohle [20 % Pd-Gehalt] bis zur Beendigung der Wasserstoffaufnahme hydriert (ca. 2 Stunden). Nach Abfiltrieren des Katalysators wird die gesamte Reaktionslösung imVakuum eingeengt. Man erhält 0,81 g (100 % der Theorie) N-Methyl-L-isoleucyl-D-lactyl-N-methyl-L-isoleucyl-D-lactyl-N-methyl-L-isoleucyl-D-milchsäure, das ohne weitere Reinigung cyclisiert werden kann.
EI-MS m/z (%): 615 (M⁺,3); 600 (1); 558 (7); 472 (8); 286(14); 100(100)

Analog können die in der nachstehenden Tabelle 2 auf führten Verbindungen der allgemeinen Formel ( II ) als LDLDLD - Stereoisomere hergestellt werden.

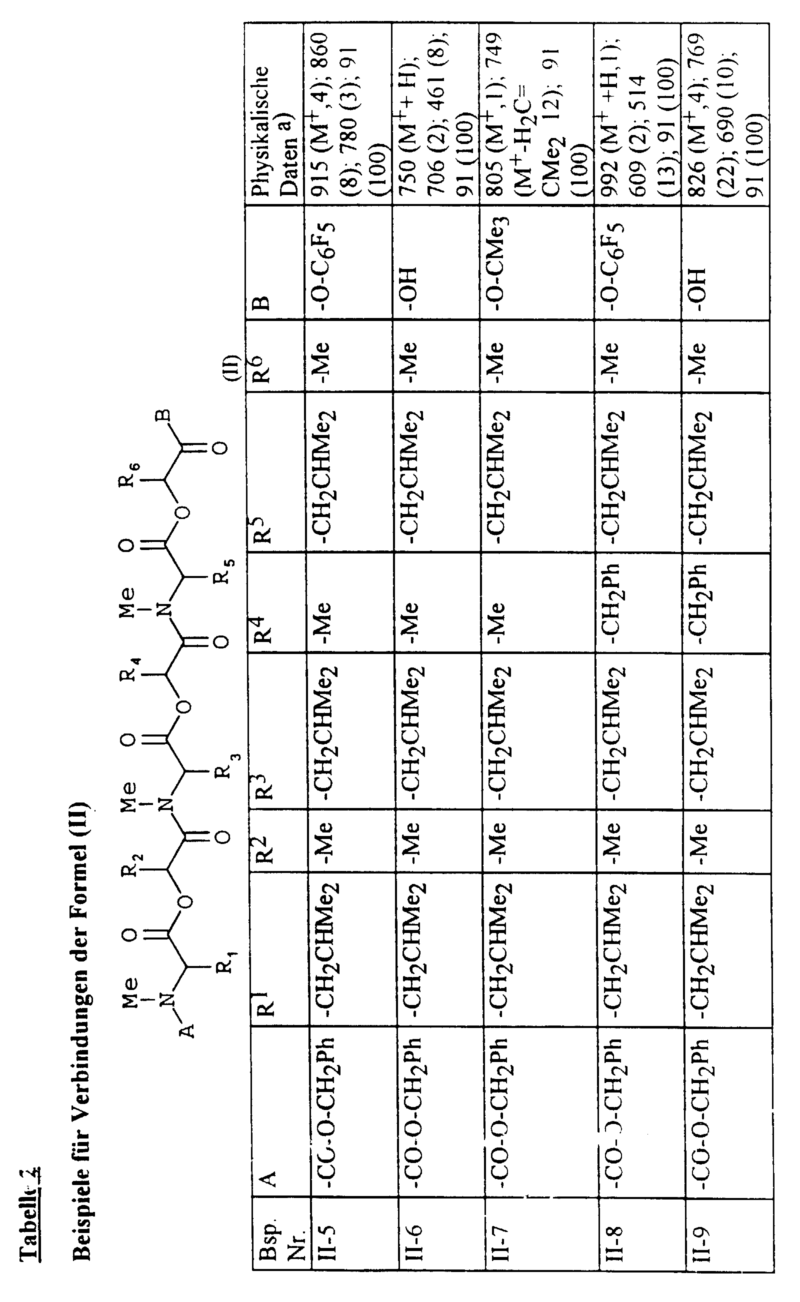

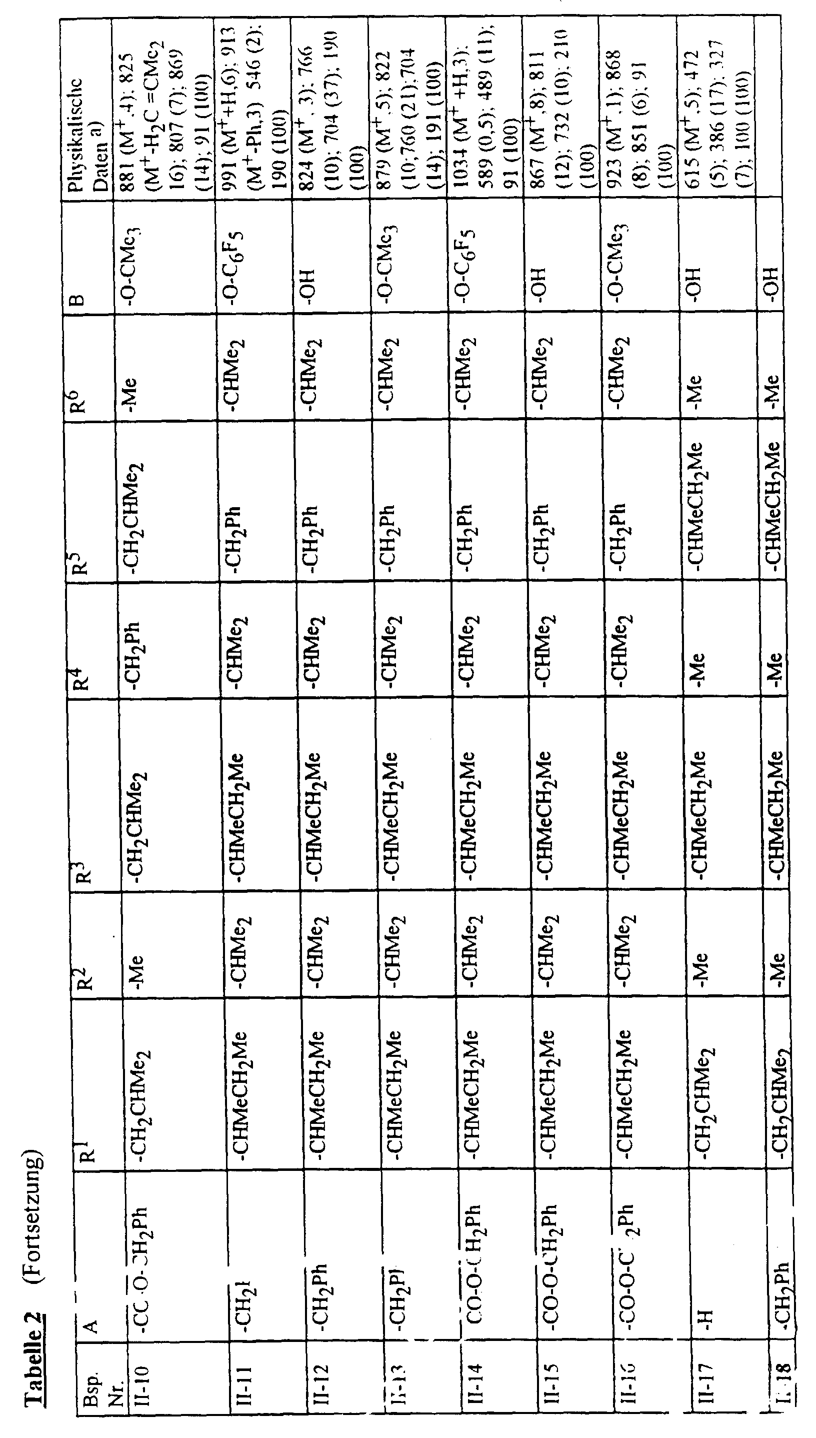

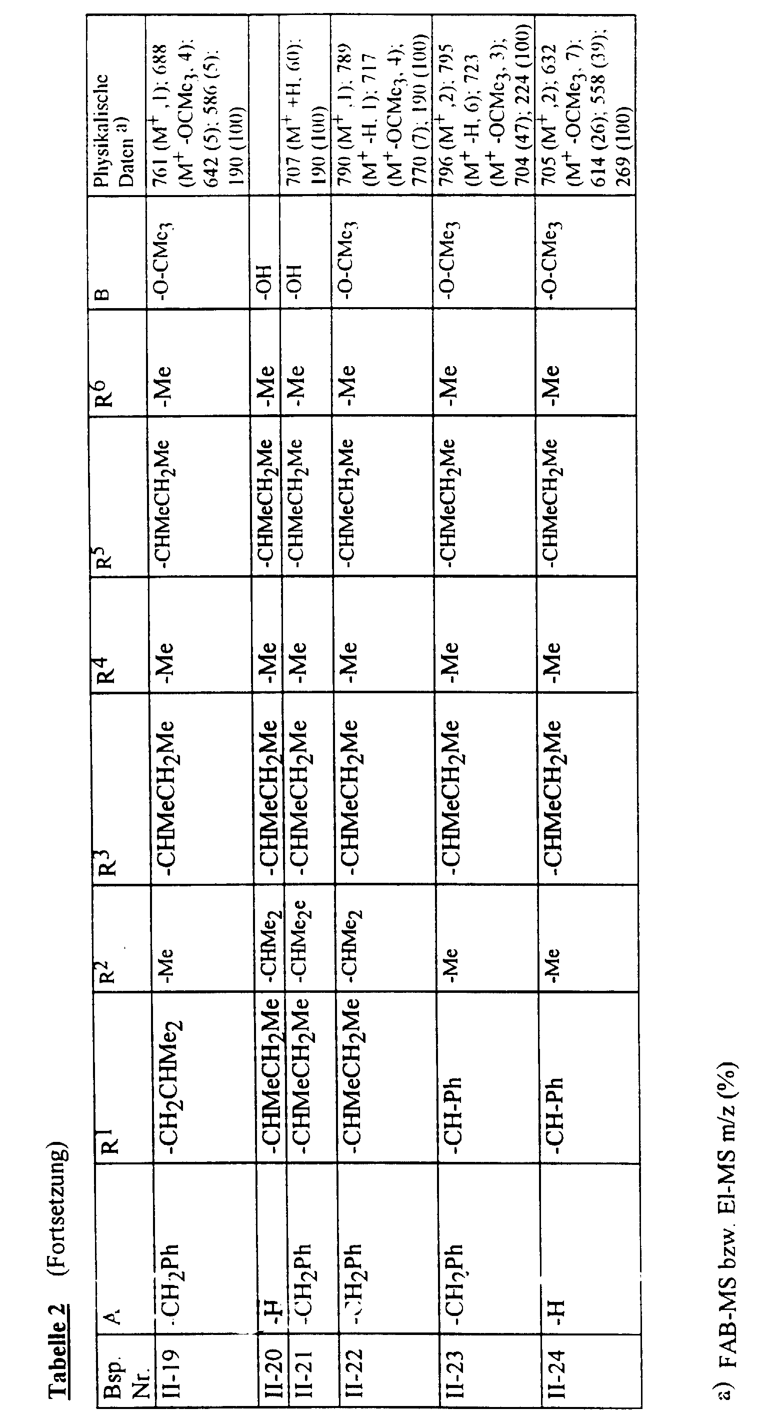

### Ausgangsstoffe der Formel (III)

### Beispiel (III-1)

### N-Benzyl-N-methyl-L-leucyl-D-lactyl-N-methyl-L-leucyl-D-milchsäure-tert.-butylester

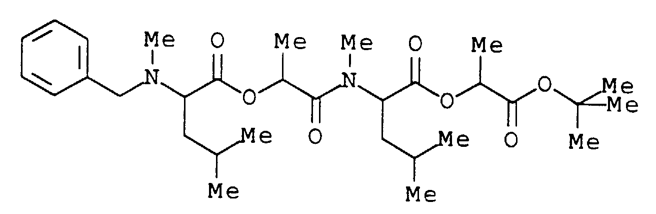

Die Kupplungsreaktion erfolgt analog der Reaktionsvorschrift des Beispiels ( II - 1 ) unter Verwendung von:
12,4 g (40,3 mMol) N-Benzyl-N-methyl-L-leucyl-D-milchsäure,
11,0 g (40,3 mMol) N-Methyl-L-leucyl-D-milchsäure-tert.-butylester,
100 ml Methylenchlorid,
11,5 g (88,7 mMol) N,N-Diisopropylethylamin ("Hünig's Base"),
11,3 g (44,3 mMol) Bis(2-oxo-3-oxazolidinyl)-phosphoniumsäurechlorid (BOP-Cl).

Das zurückbleibende Rohprodukt wird über eine Kieselgelsäule (Kieselgel 60 - Merck, Korngröße : 0,04 bis 0,063 mm) mit dem Fließmittel Toluol :
Essigsäureethylester (20:1) chromatographiert. Man erhält 21,8 g (96,0 % der Theorie) N-Benzyl-N-methyl-L-leucyl-D-lactyl-N-methyl-L-leucyl-D-milchsäure-tert.-butylester.
- EI-MS m/z (%):: 562 (M⁺,3); 489 (M⁺- OCMe₃,7); 443 (2); 387 (3); 344 (1); 190 (PhCH₂-NMe-CH-CH₂Me₂, 100); 120 (PhCH₂-NMe-, 31)

### Beispiel ( III - 2 )

### N-Methyl-L-leucyl-D-lactyl-N-methyl-L-leucyl-D-milchsäure-tert.-butylester

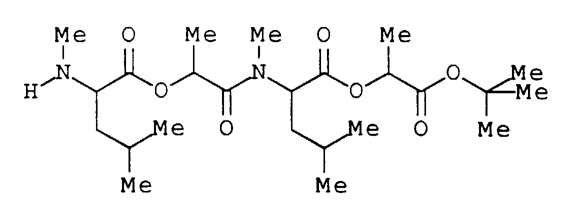

21,8 g (38,7 mMol) Bn-(-L-MeLeu-D-Lac-)₂-O-^{t}Bu werden in 300 ml Ethanol gelöst und in Gegenwart von 2,2 g Pd(OH)₂/Kohle [20 % Pd-Gehalt] bis zur Beendigung der Wasserstoffaufnahme hydriert (ca. 2,5 Stunden). Nach Abfiltrieren des Katalysators wird die gesamte Reaktionslösung imVakuum eingeengt. Man erhält 18,3 g (100 % der Theorie) N-Methyl-L-leucyl-D-lactyl-N-methyl-L-leucyl-D-milchsäure-tert.-butylester, das ohne weitere Reinigung für die Kupplungsreaktion verwendet werden kann.

EI-MS m/z (%): 472 (M⁺,4); 457 (1); 428 (1); 399 (6); 100 (HNMe-CH-CH₂Me₂, 100)

Analog können die in der nachstehenden Tabelle 3 aufgeführten Verbindungen der allgemeinen Formel ( III ) als LDLD - Stereoisomere hergestellt werden.

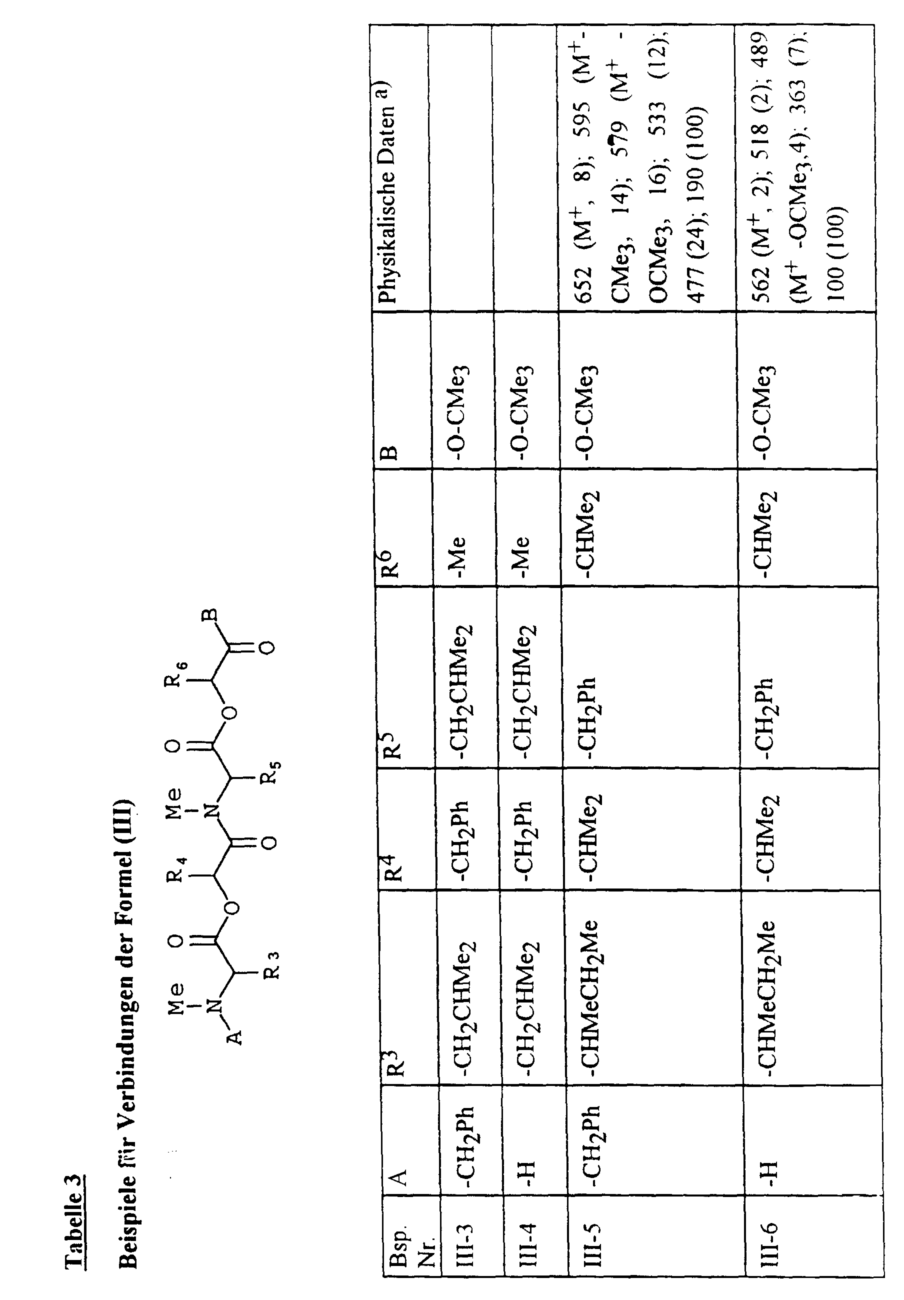

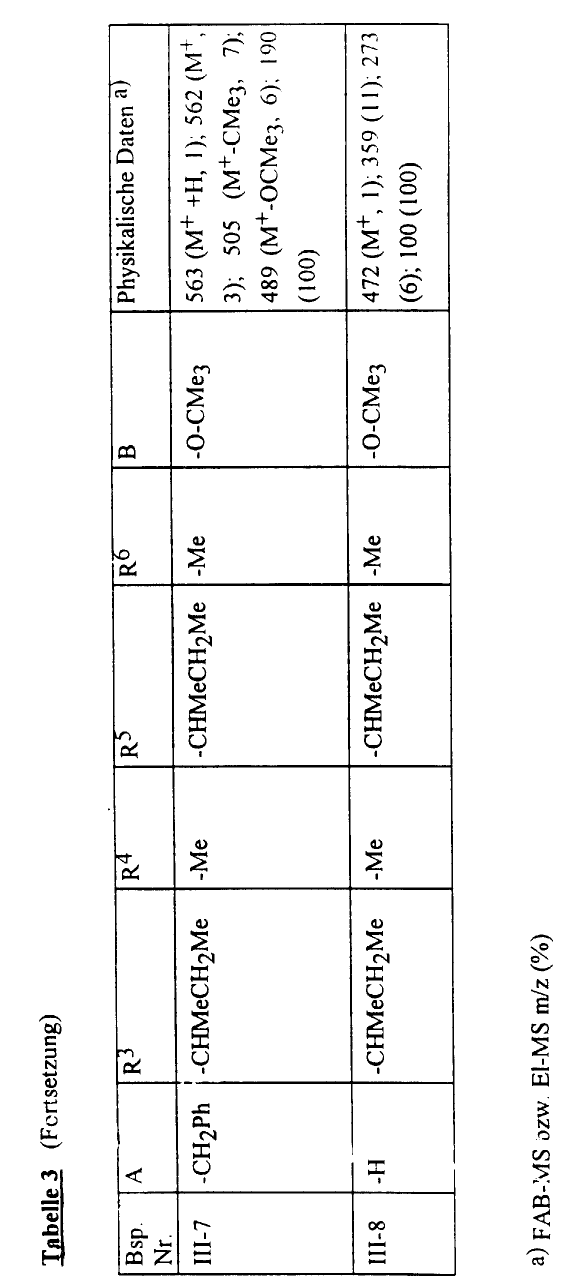

### Ausgangsstoffe der Formel (IV), (V) und (VI)

### Beispiel (IV-1)

### N-Benzyloxycarbonyl-N-methyl-L-leucyl-D-milchsäure-tert.-butylester

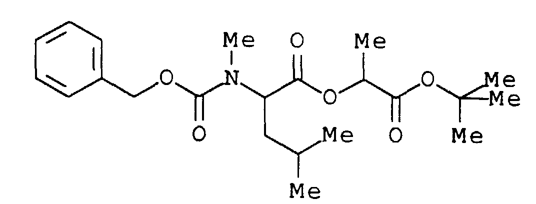

10,0 g (35,8 mMol) N-Benzyloxycarbonyl-N-methyl-leucin werden in 150 ml Methanol und 15 ml Wasser gelöst, mit 19,5 ml einer 20 %igen Cäsiumcarbonat-Lösung versetzt und ca. eine Stunde bei Raumtemperatur gerührt. Anschließend wird zweimal mit ca. 50 ml absolutem Dimethylformamid versetzt, im Vakuum eingeengt und im Hochvakuum getrocknet. Das Cäsiumsalz wird in 75 ml Dimethylformamid vorgelegt, mit 7,0 g (35,8 mMol) L-2-Chlor-milchsäure-tert.-butylester versetzt und ca. 18 Stunden bei Raumtemperatur gerührt. Die gesamte Reaktionslösung wird im Vakuum eingeengt, der ölige Rückstand in Methylenchlorid aufgenommen und zweimal mit Wasser geschüttelt. Danach wird die organische Phase abgetrennt, über Natriumsulfat getrocknet und im Vakuum eingeengt.

Das zurückbleibende Rohprodukt wird über eine Kieselgelsäule (Kieselgel 60 - Merck, Korngröße : 0,04 bis 0,063 mm) mit dem Fließmittel Toluol : Essigsäureethylester (40:1) chromatographiert. Man erhält 14,4 g (100 % der Theorie) N-Benzyloxycarbonyl-N-methyl-L-leucyl-D-milchsäure-tert.-butylester.
- EI-MS m/z (%):: 407 (M⁺,2); 351 (10); 234 (39); 387 (3); 344 (1); 190 (PhCH₂-NMe-CH-CH₂Me₂,69); 91 (PhCH₂,100)

### Beispiel (V-1)

### N-Benzyl-N-methyl-L-isoleucyl-D-hydroxyisovaleriansäure

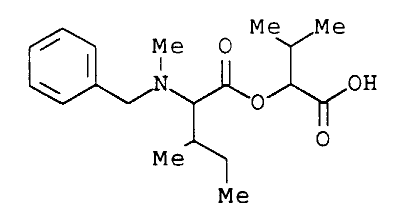

Die C-terminale Acidolyse erfolgt analog der Reaktionsvorschrift des Beispiels ( II - 2 ) unter Verwendung von:
10,5 g (26,8 mMol) N-Benzyl-N-methyl-L-isoleucyl-D-hydroxyisovaleriansäure-tert.-butylester,
250 ml Methylenchlorid.

Man erhält 8,5 g (94,5 % der Theorie) N-Benzyl-N-methyl-L-isoleucyl-D-hydroxyisovaleriansäure, das ohne weitere Reinigung weiter umgesetzt werden kann.
- EI-MS m/z (%):: 335 (M⁺,1); 278 (19); 190 (PhCH₂-NMe-CH-CHMeCH₂Me,100); 91 (PhCH₂,84)

### Beispiel (VI-1)

### N-Methyl-L-phenylalanyl-D-hydoxyvaleriansäure-tert.-butylester

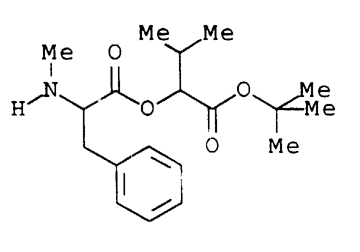

Die N-terminale Deblockierung erfolgt analog der Reaktionsvorschrift des Beispiels ( III- 2 ) unter Verwendung von:
10,0 g (23,5 mMol) N-Benzyl-N-methyl-L-phenylalanyl-D-hydroxyisovaleriansäure-tert.-butylester,
250 ml Ethanol,
1,0 g Pd(OH)₂/Kohle [20 % Pd-Gehalt]

Man erhält 7,5 g (95,2 % der Theorie) N-Methyl-L-phenylalanyl-D-hydroxyvaleriansäure-tert.-butylester, das ohne weitere Reinigung weiter umgesetzt werden kann.

¹H-NMR (400 MHz, CDCl₃, δ): 0,80; 0,85 (2d, 6H, 2 x -CH₃; J = 6,9 Hz); 1,46 (s, 9H, -C(CH₃)₃); 2,42 (s, 3H, -N-CH₃); 2,94; 2,97 (2d, 2H, -CH₂-Phe); 3,55 (m, 1H,-O-CH); 4,58 (d, 1H, -N-CH; J = 4,7 Hz); 7,18-7,26 (m, 5H, arom.-H) ppm
- EI-MS m/z (%):: 336 (M⁺+H,7); 335 (M⁺,2); 262 (M⁺- O-CMe₃,12); 188 (100); 134 (81)

Analog können die in den nachstehenden Tabellen 4, 5 und 6 aufgeführten Verbindungen der allgemeinen Formeln (IV), (V) und ( VI ) als L-D-Stereoisomere hergestellt werden.

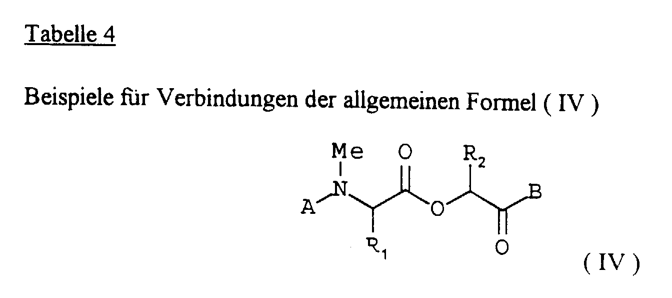

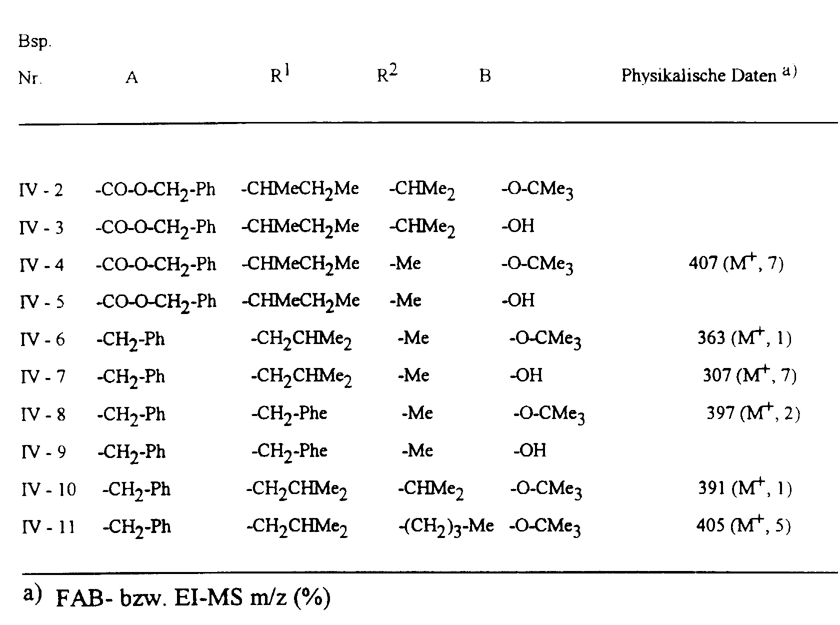

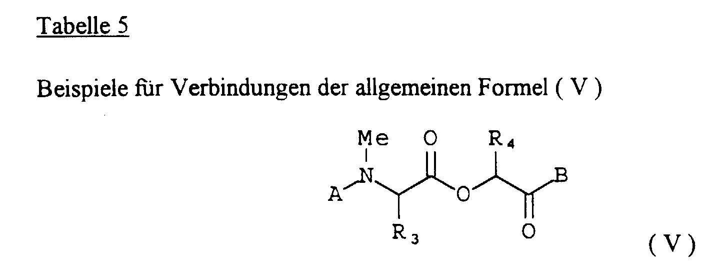

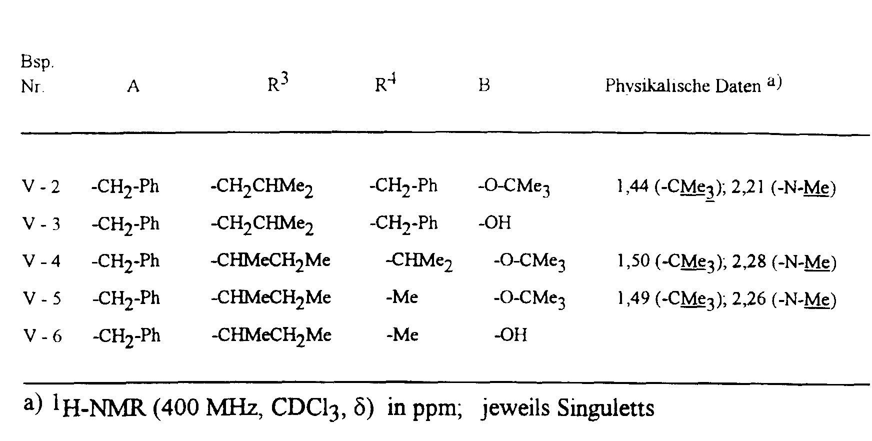

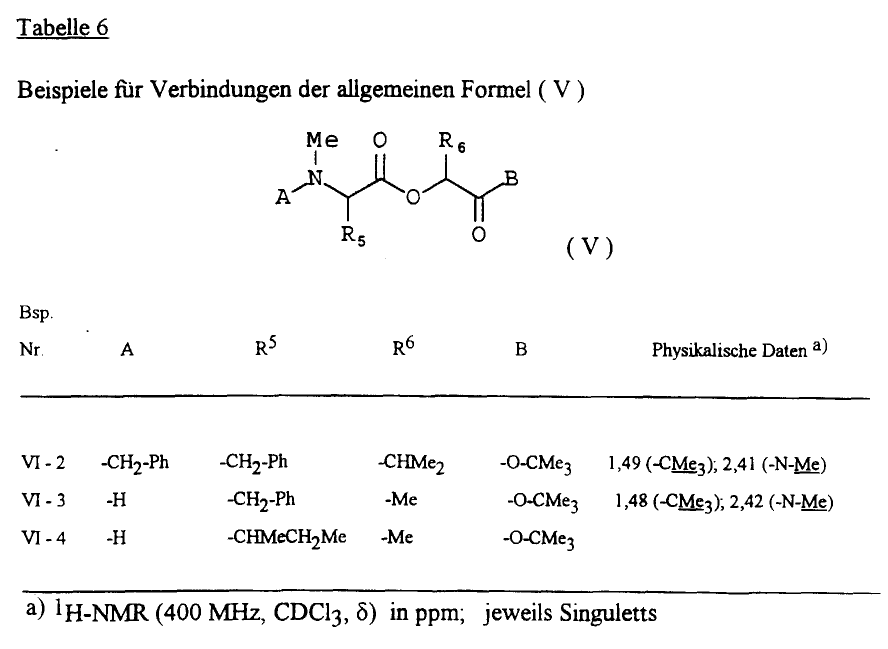

## Patentansprüche

1. Verwendung von cyclischen Depsipeptiden mit 18 Ringatomen der allgemeinen Formel (I) 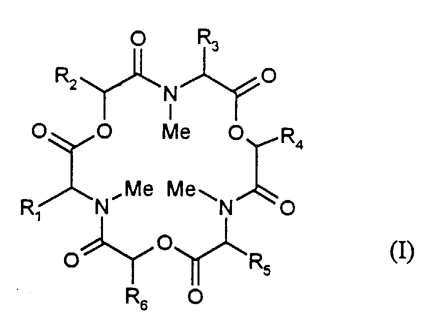 in welcher
R¹, R³ und R⁵ unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
R², R⁴ und R⁶ unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
sowie deren optische Isomere und Racemate,
zur Herstellung von Mitteln zur Bekämpfung von Endoparasiten in der Medizin und Tiermedizin.

2. Cyclische Depsipeptide mit 18 Ringatomen der allgemeinen Formel (Ia) 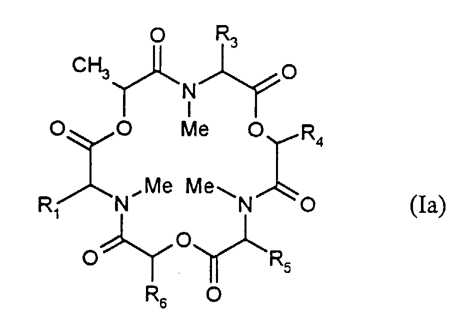 in welcher
R¹ für geradkettiges oder verzweigtes Alkyl mit 2 bis 8 Kohlenstoffatomen oder Cycloalkylalkyl steht,
R³ und R⁵ unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
R⁴ und R⁶ unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
sowie deren optische Isomere und Racemate, ausgenommen die Verbindung der Formel (Ia), in welcher die Reste R¹, R³, R⁴, R⁵, R⁶ für i-Propyl stehen.

3. Verfahren zur Herstellung von cyclischen Depsipeptiden mit 18 Ringatomen der allgemeinen Formel (Ia) gemäß Anspruch 2 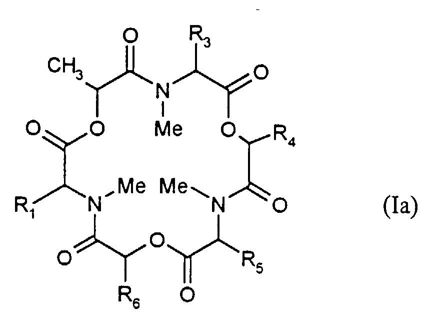 in welcher
R¹ für geradkettiges oder verzweigtes Alkyl mit 2 bis 8 Kohlenstoffatomen oder Cycloalkylalkyl steht,
R³ und R⁵ unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
R⁴ und R⁶ unabhängig voneinander für geradkettiges oder verzweigtes Alkyl mit bis zu 8 Kohlenstoffatomen, Cycloalkylalkyl sowie gegebenenfalls substituiertes Phenyl-C₁-C₄-alkyl, wobei als Substituenten genannt seien Halogen, Hydroxy, Alkyl, Alkoxy, stehen,
dadurch gekennzeichnet, daß man
a) carboxy-aktivierte offenkettige Hexadepsipeptide der allgemeinen Formel (IIc) 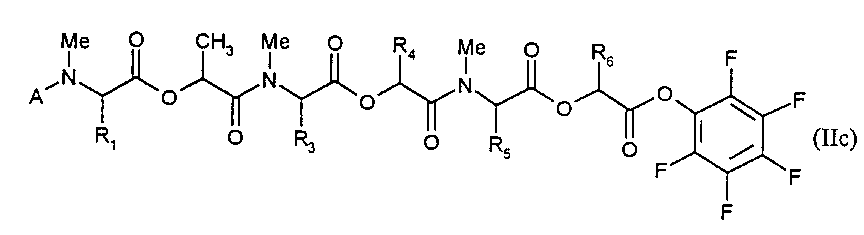 in welcher
A für eine gegenüber der Aktivesterschutzgruppe selektiv entfernbare Aminoschutzgruppe, wie Benzyl oder Benzyloxycarbonyl, steht und
R¹, R³, R⁴, R⁵ und R⁶ die oben angegebene Bedeutung haben,
in Gegenwart eines Hydrierkatalysators, in Gegenwart eines basischen Reaktionshilfsmittels und in Gegenwart eines Verdünnungsmittels cyclisiert, oder
b) offenkettige Hexadepsipeptide der allgemeinen Formel (IId) 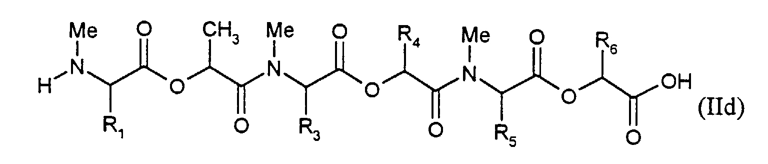 in welcher
R¹, R³, R⁴, R⁵ und R⁶ die oben angegebene Bedeutung haben,
in Gegenwart eines Kupplungsreagenzes, in Gegenwart eines basischen Reaktionshilfsmittels und in Gegenwart eines Verdünnungsmittels cyclisiert.

## Claims

1. Use of cyclic depsipeptides having 18 ring atoms of the general formula (I) 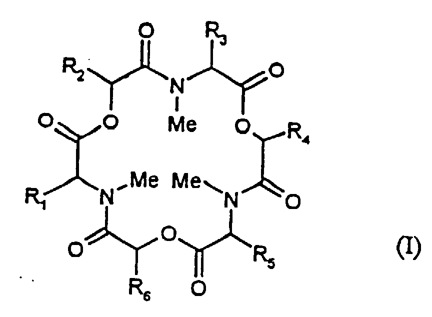 in which
R¹, R³ and R⁵ independently of one another represent straight-chain or branched alkyl having up to 8 carbon atoms, cycloalkylalkyl and optionally substituted phenyl-C₁-C₄-alkyl, substituents which may be mentioned being halogen, hydroxyl, alkyl or alkoxy,
R² , R⁴ and R⁶ independently of one another represent straight-chain or branched alkyl having up to 8 carbon atoms, cycloalkylalkyl and optionally substituted phenyl-C₁-C₄-alkyl, substituents which may be mentioned being halogen, hydroxyl, alkyl or alkoxy,
and their optical isomers and racemates,
in medicine and veterinary medicine for the preparation of compositions for combating endoparasites.

2. Cyclic depsipeptides having 18 ring atoms of the general formula (Ia) 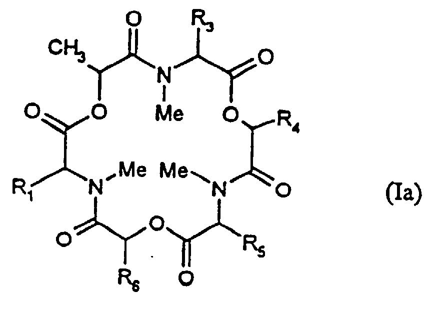 in which
R¹ represents straight-chain or branched alkyl having 2 to 8 carbon atoms or cycloalkylalkyl
R³ and R⁵ independently of one another represent straight-chain or branched alkyl having up to 8 carbon atoms, cycloalkylalkyl and optionally substituted phenyl-C₁-C₄-alkyl, substituents which may be mentioned being halogen, hydroxyl, alkyl or alkoxy,
R⁴ and R⁶ independently of one another represent straight-chain or branched alkyl having up to 8 carbon atoms, cycloalkylalkyl and optionally substituted phenyl-C₁-C₄-alkyl, substituents which may be mentioned being halogen, hydroxyl, alkyl or alkoxy,
and their optical isomers and racemates, except for the compound of the formula (Ia) in which the radicals R¹, R³, R⁴, R⁵ and R⁶ represent i-propyl.

3. Process for the preparation of cyclic depsipeptides having 18 ring atoms of the general formula (Ia) according to Claim 2 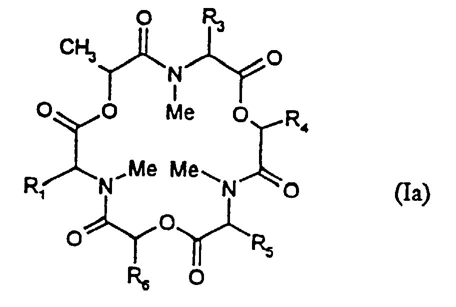 in which
R¹ represents straight-chain or branched alkyl having 2 to 8 carbon atoms cycloalkylalkyl,
R³ and R⁵ independently of one another represent straight-chain or branched alkyl having up to 8 carbon atoms, cycloalkylalkyl and optionally substituted phenyl-C₁-C₄-alkyl, substituents which may be mentioned being halogen, hydroxyl, alkyl or alkoxy,
R⁴ and R⁶ independently of one another represent straight-chain or branched alkyl having up to 8 carbon atoms, cycloalkylalkyl and optionally substituted phenyl-C₁-C₄-alkyl, substituents which may be mentioned being halogen, hydroxyl, alkyl or alkoxy,
characterized in that
a) carboxyl-activated open-chain hexadepsipeptides of the general formula (IIc) 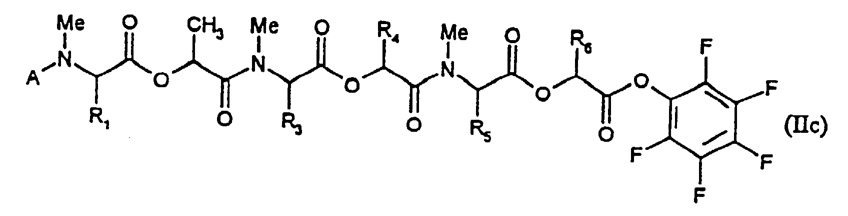 in which
A represents an amino protective group which can be detached selectively with regard to the active ester protecting group, such as benzyl or benzyloxycarbonyl, and
R¹, R³, R⁴, R⁵ and R⁶ have the abovementioned meaning,
are cyclized in the presence of a hydrogenation catalyst, in the presence of a basic reaction auxiliary and in the presence of a diluent, or
b) open-chain hexadepsipeptides of the general formula (IId) 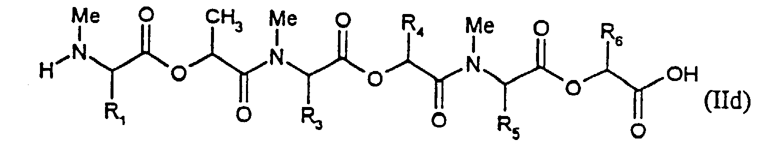 in which
R¹, R³, R⁴, R⁵ and R⁶ have the abovementioned meaning,
are cyclized in the presence of a coupling reagent, in the presence of a basic reaction auxiliary and in the presence of a diluent.

## Revendications

1. Utilisation de depsipeptides cycliques à noyau de 18 atomes, de formule générale (I) 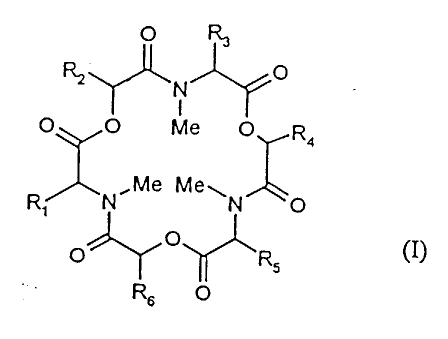 dans laquelle
R¹, R³ et R⁵ représentent indépendamment l'un de l'autre un groupe alkyle linéaire ou ramifié ayant jusqu'à 8 atomes de carbone, un groupe cycloalkylalkyle ainsi qu'un groupe phényl-(alkyle en C₁ à C₄) éventuellement substitué, et on mentionne alors comme substituants un halogène, un groupe hydroxy, alkyle, alkoxy,
R², R⁴ et R⁶ représentent indépendamment l'un de l'autre un groupe alkyle linéaire ou ramifié ayant jusqu'à 8 atomes de carbone, cycloalkylalkyle ainsi que phényl-(alkyle en C₁ à C₄) éventuellement substitué, et on mentionne alors comme substituants un halogène, un groupe hydroxy, alkyle, alkoxy,
ainsi que de leurs isomères optiques et de leurs racémates, pour la préparation de compositions destinées à combattre des endoparasites en médecine humaine et en médecine vétérinaire.

2. Depsipeptides cycliques à noyau de 18 atomes de formule générale (Ia) 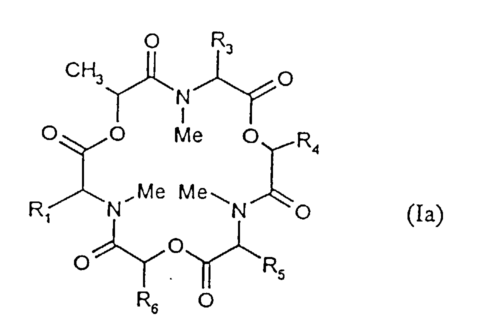 dans laquelle
R¹ est un groupe alkyle linéaire ou ramifié ayant 2 à 8 atomes de carbone ou un groupe cycloalkylalkyle,
R³ et R⁵ représentent indépendamment l'un de l'autre un groupe alkyle linéaire ou ramifié ayant jusqu'à 8 atomes de carbone, un groupe cycloalkylalkyle ainsi qu'un groupe phényl-(alkyle en C₁ à C₄) éventuellement substitué, et on mentionne alors comme substituants un halogène, un groupe hydroxy, alkyle, alkoxy,
R⁴ et R⁶ représentent indépendamment l'un de l'autre un groupe alkyle linéaire ou ramifié ayant jusqu'à 8 atomes de carbone, un groupe cycloalkylalkyle ainsi qu'un groupe phényl-(alkyle en C₁ à C₄) éventuellement substitué, et on mentionne alors comme substituants un halogène, un groupe hydroxy, alkyle, alkoxy,
ainsi que leurs isomères optiques et leurs racémates, à l'exception du composé de formule (Ia) dans laquelle les restes R¹, R³, R⁴, R⁵, R⁶ représentent un groupe isopropyle.

3. Procédé de production de depsipeptides cycliques à noyau de 18 atomes de formule générale (Ia) suivant la revendication 2 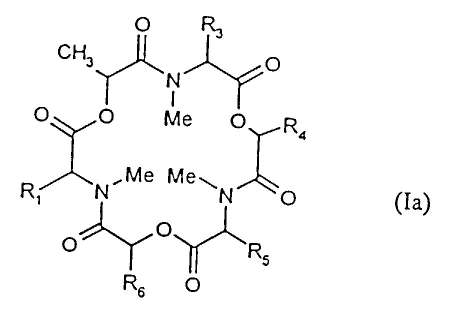 dans laquelle
R¹ est un groupe alkyle linéaire ou ramifié ayant 2 à 8 atomes de carbone ou un groupe cycloalkylalkyle,
R³ et R⁵ représentent indépendamment l'un de l'autre un groupe alkyle linéaire ou ramifié ayant jusqu'à 8 atomes de carbone, un groupe cycloalkylalkyle ainsi qu'un groupe phényl-(alkyle en C₁ à C₄) éventuellement substitué, et on mentionne alors comme substituants un halogène, un groupe hydroxy, alkyle, alkoxy,
R⁴ et R⁶ représentent indépendamment l'un de l'autre un groupe alkyle linéaire ou ramifié ayant jusqu'à 8 atomes de carbone, cycloalkylalkyle ainsi que phényl-(alkyle en C₁ à C₄) éventuellement substitué, et on mentionne alors comme substituants un halogène, un groupe hydroxy, alkyle, alkoxy,
caractérisé en ce que
a) on cyclise des hexadepsipeptides à chaîne ouverte carboxy-activés de formule générale (IIc) 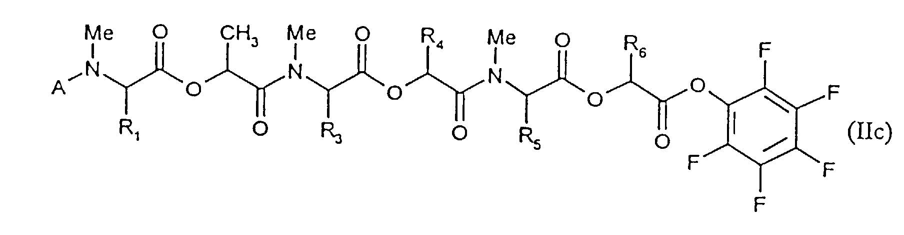 dans laquelle
A représente un groupe protégeant la fonction amino, tel que benzyle ou benzyloxycarbonyle, sélectivement éliminable par rapport au groupe protecteur ester actif et
R¹, R³, R⁴, R⁵ et R⁶ ont la définition indiquée ci-dessus, en présence d'un catalyseur d'hydrogénation, en
présence d'une substance auxiliaire basique de réaction et en présence d'un diluant,
ou bien
b) on cyclise des hexadepsipeptides à chaîne ouverte de formule générale (IId) 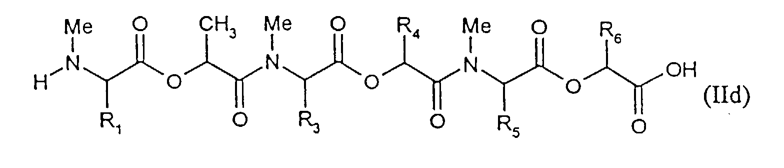 dans laquelle
R¹, R³, R⁴, R⁵ et R⁶ ont la définition indiquée ci-dessus, en présence d'un réactif de couplage, en présence
d'une substance auxiliaire basique de réaction et en présence d'un diluant.
